# EUROPEAN PATENT APPLICATION

(11) **EP 4 207 207 A1**
(43) Date of publication of application: **05.07.2023**
(21) Application number: 22217098.7
(22) Date of filing: 29.12.2022
(51) Int. Cl.: G16H 10/20, G16H 20/70, G16H 50/30, G16H 50/70

(54) **PERSONALIZED THERAPY DELIVERY VIA ASSESSMENT TRACKING**

(30) Priority: 30.12.2021 US 202163294939 P; 30.12.2021 US 202163294941 P; 30.12.2021 US 202163294946 P; 30.12.2021 US 202163294975 P; 30.12.2021 US 202163294980 P; 30.12.2021 US 202163294986 P
(71) Applicant: Woebot Labs, Inc., San Francisco, CA 94107 (US)
(72) Inventor: Darcy, Alison, San Francisco (US); Goodwin, Kim, San Francisco (US); Gallagher, Joe, San Francisco (US); Sackett, Casey, San Francisco (US)
(74) Representative: Trichard, Louis

(57) **Abstract**

A therapy platform can leverage mood tracking to personalize delivery of therapy. User input data is collected to generate one or more mood scores that change over time. Tracked mood scores can include an overall mood score (e.g., -100 to 100 where -100 represents a strongly negative or "bad" mood and 100 represents a strongly positive or "happy" mood) and/or enumerated mood scores (e.g., numerical scores tracking individual, enumerated moods, such as "anxious," "sad," "tired," "happy," and the like). In some cases, an overall mood score can be made up of enumerated mood scores. Tracked mood scores can be used to inform the selection of what therapy to provide. For example, a first therapy tool may be selected for delivery when the user evidences a first set of mood scores, whereas a second therapy tool may be selected when the user evidences a second set of mood scores.

## Description

### CROSS-REFERENCE TO RELATED APPLICATIONS

The present application claims the benefit of U.S. Provisional Application No. 63/294,939 filed December 30, 2021 and entitled "PERSONALIZED THERAPY DELIVERY VIA MOOD TRACKING," and also claims the benefit of U.S. Provisional Application No. 63/294,941 filed December 30, 2021 and entitled "PERSONALIZED THERAPY SEQUENCING VIA MOOD TRACKING," and also claims the benefit of U.S. Provisional Application no. 63/294,946 FILED December 30, 2021 and entitled "PERSONALIZED THERAPY TIMING VIA MOOD TRACKING," and also claims the benefit of U.S. Provisional Application No. 63/294,975 filed December 30, 2021 and entitled "PERSONALIZED THERAPY DELIVERY VIA ASSESSMENT TRACKING," and also claims the benefit of U.S. Provisional Application No. 63/294,980 filed December 30, 2021 and entitled "PERSONALIZED THERAPY SEQUENCING VIA ASSESSMENT TRACKING," and also claims the benefit of U.S. Provisional Application no. 63/294,986 FILED December 30, 2021 and entitled "PERSONALIZED THERAPY TIMING VIA ASSESSMENT TRACKING."

### TECHNICAL FIELD

The present disclosure relates to therapy devices generally and more specifically to providing therapy that is personalized to a user's needs.

### BACKGROUND

Mental health problems are widespread, persistent, and stigmatized, with depression now the leading cause of disability worldwide. There are not enough trained professionals to deliver appropriate screening, diagnosis, and treatment. It can be difficult for individuals suffering from mental health problems to obtain treatment for a number of reasons, including difficulty in finding a provider, difficulty in paying for treatment, difficulty in self-identifying a need for treatment, and other reasons. Even if an individual finds a suitable provider and makes an effort to obtain treatment, that individual may need to wait long periods of time before an initial session due to the provider's schedule. Additionally, an individual's success in certain types of therapy, such as Cognitive-behavioral Therapy (CBT), depends strongly on the individual's ability to continue practicing certain exercises and data collection between sessions.

Self-directed treatment methods generally involve following exercises provided by a provider or presented in a book. Certain chatbots exist to mimic human-to-human therapy or assist a user in following self-directed treatment, but such chatbots follow fixed scripts, are unable to adapt to a user's individual needs, and are unable to establish a strong therapeutic bond like those established between patient and human provider.

There is a need for a tool to provide automated, personalized therapy to individuals. There is a need for a tool that can facilitate self-directed treatment in a fashion that is personalized to the individual. There is a need for a tool that can provide personalized therapy to individuals that dynamically changes according to the needs of the individual.

### SUMMARY

The term embodiment and like terms are intended to refer broadly to all of the subject matter of this disclosure and the claims below. Statements containing these terms should be understood not to limit the subject matter described herein or to limit the meaning or scope of the claims below. Embodiments of the present disclosure covered herein are defined by the claims below, supplemented by this summary. This summary is a high-level overview of various aspects of the disclosure and introduces some of the concepts that are further described in the Detailed Description section below. This summary is not intended to identify key or essential features of the claimed subject matter, nor is it intended to be used in isolation to determine the scope of the claimed subject matter. The subject matter should be understood by reference to appropriate portions of the entire specification of this disclosure, any or all drawings and each claim.

Embodiments of the present disclosure include a computer-implemented method, comprising: receiving cohort information associated with a user; receiving mood information associated with the user, wherein receiving the mood information includes receiving user input data and generating one or more mood scores based at least in part on the input data; accessing a mood-based personalization model based on the cohort information, wherein the mood-based personalization model is trained, at least in part, using training data for a plurality individuals associated with the cohort, wherein the training data includes a plurality of mood scores associated with the plurality of individuals associated with the cohort; determining a therapy tool to be used by applying the one or more mood scores to the mood-based personalization model; and facilitating providing personalized therapy to the user using the determined therapy tool.

Embodiments of the present disclosure include a computer-implemented method, comprising: receiving cohort information associated with a user; receiving mood information associated with the user, wherein receiving the mood information includes receiving user input data and generating one or more mood scores based at least in part on the input data; accessing a mood-based personalization model based on the cohort information, wherein the mood-based personalization model is trained, at least in part, using training data for a plurality individuals associated with the cohort, wherein the training data includes a plurality of mood scores associated with the plurality of individuals associated with the cohort; determining therapy sequencing to be used by applying the one or more mood scores to the mood-based personalization model, wherein the determined therapy sequencing is indicative of i) an order for applying a plurality of therapy tools; ii) an order for addressing therapy targets; or iii) a combination of i and ii; and facilitating providing personalized therapy to the user using the determined therapy sequencing.

Embodiments of the present disclosure include a computer-implemented method, comprising: receiving cohort information associated with a user; receiving mood information associated with the user, wherein receiving the mood information includes receiving user input data and generating one or more mood scores based at least in part on the input data; accessing a mood-based personalization model based on the cohort information, wherein the mood-based personalization model is trained, at least in part, using training data for a plurality individuals associated with the cohort, wherein the training data includes a plurality of mood scores associated with the plurality of individuals associated with the cohort; determining therapy timing to be used by applying the one or more mood scores to the mood-based personalization model, wherein the determined therapy timing is indicative of i) a frequency for applying one or more therapy tools; ii) a future time to apply the one or more therapy tools; or iii) a combination of i and ii; and facilitating providing personalized therapy to the user using the determined therapy timing.

Embodiments of the present disclosure include a computer-implemented method, comprising: receiving first user input data associated with a therapy target; generating one or more first assessment scores based at least in part on the first user input data, wherein the one or more first assessment scores are indicative of i) a pre-therapy perceived severity of the therapy target; ii) a pre-therapy perceived severity of a condition associated with the therapy target; or iii) a combination of i and ii; providing, after receiving the first user input data, therapy to a user using a therapy tool during a therapy session; receiving second user input data associated with the therapy target, wherein receiving the second user input data occurs after providing the therapy; generating one or more second assessment scores based at least in part on the second user input data, wherein the one or more second assessment scores are indicative of i) a post-therapy perceived severity of the therapy target; ii) a post-therapy perceived severity of the condition associated with the therapy target; or iii) a combination of i and ii; training an assessment-based personalization model based at least in part on the one or more first assessment scores, the one or more second assessment scores, and the provided therapy; determining a subsequent therapy tool to be used during a subsequent therapy session associated with the therapy target, wherein determining the subsequent therapy tool is based at least in part on the trained assessment-based personalization model; and facilitating providing personalized therapy to the user using the determined therapy tool.

Embodiments of the present disclosure include a computer-implemented method, comprising: receiving first user input data associated with a therapy target; generating one or more first assessment scores based at least in part on the first user input data, wherein the one or more first assessment scores are indicative of i) a pre-therapy perceived severity of the therapy target; ii) a pre-therapy perceived severity of a condition associated with the therapy target; or iii) a combination of i and ii; providing, after receiving the first user input data, therapy to a user using a therapy tool during a therapy session; receiving second user input data associated with the therapy target, wherein receiving the second user input data occurs after providing the therapy; generating one or more second assessment scores based at least in part on the second user input data, wherein the one or more second assessment scores are indicative of i) a post-therapy perceived severity of the therapy target; ii) a post-therapy perceived severity of the condition associated with the therapy target; or iii) a combination of i and ii; training an assessment-based personalization model based at least in part on the one or more first assessment scores, the one or more second assessment scores, and the provided therapy; determining therapy sequencing to be used during one or more subsequent therapy sessions associated with the therapy target, wherein determining the therapy sequencing is based at least in part on the trained assessment-based personalization model, and wherein the determined therapy sequencing is indicative of i) an order for applying a plurality of therapy tools; ii) an order for addressing a plurality of therapy targets including the therapy target; or iii) a combination of i and ii; and facilitating providing personalized therapy to the user using the determined therapy sequencing.

Embodiments of the present disclosure include a computer-implemented method, comprising: receiving first user input data associated with a therapy target; generating one or more first assessment scores based at least in part on the first user input data, wherein the one or more first assessment scores are indicative of i) a pre-therapy perceived severity of the therapy target; ii) a pre-therapy perceived severity of a condition associated with the therapy target; or iii) a combination of i and ii; providing, after receiving the first user input data, therapy to a user using a therapy tool during a therapy session; receiving second user input data associated with the therapy target, wherein receiving the second user input data occurs after providing the therapy; generating one or more second assessment scores based at least in part on the second user input data, wherein the one or more second assessment scores are indicative of i) a post-therapy perceived severity of the therapy target; ii) a post-therapy perceived severity of the condition associated with the therapy target; or iii) a combination of i and ii; training an assessment-based personalization model based at least in part on the one or more first assessment scores, the one or more second assessment scores, and the provided therapy; determining therapy timing to be used for one or more subsequent therapy sessions associated with the therapy target, wherein determining the therapy timing is based at least in part on the trained assessment-based personalization model, and wherein the therapy timing is indicative of i) a frequency for applying one or more therapy tools; ii) a future time to apply the one or more therapy tools; or iii) a combination of i and ii; facilitating providing personalized therapy to the user using the determined therapy timing.

Embodiments of the present disclosure include a system comprising one or more data processors; and a non-transitory computer-readable storage medium containing instructions which, when executed on the one or more data processors, cause the one or more data processors to perform the above method(s).

Embodiments of the present disclosure include a computer-program product tangibly embodied in a non-transitory machine-readable storage medium, including instructions configured to cause a data processing apparatus to perform the above method(s).

### BRIEF DESCRIPTION OF THE DRAWINGS

The specification makes reference to the following appended figures, in which use of like reference numerals in different figures is intended to illustrate like or analogous components.
FIG. 1 is a schematic diagram depicting a computing environment, according to certain aspects of the present disclosure.
FIG. 2 is a flowchart depicting a process for providing personalized therapy, according to certain aspects of the present disclosure.
FIG. 3 is a flowchart depicting a process for training a mood-based personalization model, according to certain aspects of the present disclosure.
FIG. 4 is a flowchart depicting a process for training an assessment-based personalization model, according to certain aspects of the present disclosure.
FIG. 5 is a schematic diagram depicting an example conversation path for obtaining mood information on a graphical user interface, according to certain aspects of the present disclosure.
FIG. 6 is a schematic diagram depicting an example conversation path on a graphical user interface for a depressed response, according to certain aspects of the present disclosure.
FIG. 7 is a schematic diagram depicting an example conversation path on a graphical user interface for a happy response, according to certain aspects of the present disclosure.
FIG. 8 is a chart depicting how an assessment score affects therapy timing over time, according to certain aspects of the present disclosure.
FIG. 9 is a block diagram depicting an example system architecture for implementing certain features and processes of the present disclosure.

### DETAILED DESCRIPTION

Certain aspects and features of the present disclosure relate to a therapy platform that can leverage mood tracking to personalize delivery of therapy. User input data is collected to generate one or more mood scores that change over time. Tracked mood scores can include an overall mood score (e.g., -100 to 100 where -100 represents a strongly negative or "bad" mood and 100 represents a strongly positive or "happy" mood) and/or enumerated mood scores (e.g., numerical scores tracking individual, enumerated moods, such as "anxious," "sad," "tired," "happy," and the like). In some cases, an overall mood score can be made up of enumerated mood scores. Tracked mood scores can be used to inform the selection of what therapy to provide. For example, a first therapy tool may be selected for delivery when the user evidences a first set of mood scores, whereas a second therapy tool may be selected when the user evidences a second set of mood scores.

Certain aspects and features of the present disclosure relate to a therapy platform that can leverage mood tracking to personalize sequencing of therapy. User input data is collected to generate one or more mood scores that change over time. Tracked mood scores can include an overall mood score (e.g., -100 to 100 where -100 represents a strongly negative or "bad" mood and 100 represents a strongly positive or "happy" mood) and/or enumerated mood scores (e.g., numerical scores tracking individual, enumerated moods, such as "anxious," "sad," "tired," "happy," and the like). In some cases, an overall mood score can be made up of enumerated mood scores. Tracked mood scores can be used to inform the sequencing of therapy provided to the user. For example, based on a user's mood scores, a first therapy may be provided to achieve a desired change a first mood score prior to providing a second therapy designed to achieve a desired change in a second mood score.

Certain aspects and features of the present disclosure relate to a therapy platform that can leverage mood tracking to personalize timing of therapy delivery. User input data is collected to generate one or more mood scores that change over time. Tracked mood scores can include an overall mood score (e.g., -100 to 100 where -100 represents a strongly negative or "bad" mood and 100 represents a strongly positive or "happy" mood) and/or enumerated mood scores (e.g., numerical scores tracking individual, enumerated moods, such as "anxious," "sad," "tired," "happy," and the like). In some cases, an overall mood score can be made up of enumerated mood scores. Tracked mood scores can be used to inform the timing (e.g., delivery time and/or frequency) of when therapy is delivered to an individual to achieve the desired therapeutic effect and/or to make or avoid a certain change in mood score(s) (e.g., provide therapy at a timing that will not decrease the user's overall mood score).

Certain aspects and features of the present disclosure relate to a therapy platform that can leverage mood tracking to personalize delivery of therapy. User input data is collected to generate one or more mood scores that change over time. Tracked mood scores can include an overall mood score (e.g., -100 to 100 where -100 represents a strongly negative or "bad" mood and 100 represents a strongly positive or "happy" mood) and/or enumerated mood scores (e.g., numerical scores tracking individual, enumerated moods, such as "anxious," "sad," "tired," "happy," and the like). In some cases, an overall mood score can be made up of enumerated mood scores. Tracked mood scores can be used to inform the selection of what therapy to provide. For example, a first therapy tool may be selected for delivery when the user evidences a first set of mood scores, whereas a second therapy tool may be selected when the user evidences a second set of mood scores.

Certain aspects and features of the present disclosure relate to a therapy platform that can leverage mood tracking to personalize sequencing of therapy. User input data is collected to generate one or more mood scores that change over time. Tracked mood scores can include an overall mood score (e.g., -100 to 100 where -100 represents a strongly negative or "bad" mood and 100 represents a strongly positive or "happy" mood) and/or enumerated mood scores (e.g., numerical scores tracking individual, enumerated moods, such as "anxious," "sad," "tired," "happy," and the like). In some cases, an overall mood score can be made up of enumerated mood scores. Tracked mood scores can be used to inform the sequencing of therapy provided to the user. For example, based on a user's mood scores, a first therapy may be provided to achieve a desired change a first mood score prior to providing a second therapy designed to achieve a desired change in a second mood score.

Certain aspects and features of the present disclosure relate to a therapy platform that can leverage mood tracking to personalize timing of therapy delivery. User input data is collected to generate one or more mood scores that change over time. Tracked mood scores can include an overall mood score (e.g., -100 to 100 where -100 represents a strongly negative or "bad" mood and 100 represents a strongly positive or "happy" mood) and/or enumerated mood scores (e.g., numerical scores tracking individual, enumerated moods, such as "anxious," "sad," "tired," "happy," and the like). In some cases, an overall mood score can be made up of enumerated mood scores. Tracked mood scores can be used to inform the timing (e.g., delivery time and/or frequency) of when therapy is delivered to an individual to achieve the desired therapeutic effect and/or to make or avoid a certain change in mood score(s) (e.g., provide therapy at a timing that will not decrease the user's overall mood score).

Aspects and features of the present disclosure include a therapy-providing system capable of providing therapy to a user, such as to monitor, diagnose, and/or treat mental health disorders. While aspects and features of the present disclosure can be used in various environments and for various purposes, the present disclosure can be especially useful when implemented as an artificial intelligence chat-based tool, commonly known as a chatbot. Certain aspects and features of the present disclosure, when implemented with a chat-based tool, provide a quick and easy way for a user to obtain therapy on demand and/or when the therapy may be best helpful (e.g., when the user is in a mood especially receptive to receiving certain therapy and/or at certain times or frequencies that render the therapy especially effective for the user).

With traditional, human therapy providers, an individual only receives treatment from the provider during scheduled sessions or, if permitted, urgent sessions (e.g., urgent calls). Between these sessions, the only treatment received by the individual is entirely self-directed, such as a user practicing exercises provided by the provider at a prior session. Such self-directed treatment is dependent on the user remembering to do the treatment, being motivated to do the treatment, doing the treatment correctly, and recording or remembering the results of the treatment.

Because the provider experiences limited interactions with the individual, the provider may only interact with the individual when that individual is experiencing certain select moods, and may never interact with the individual when that individual is experiencing other moods. For example, an individual who regularly sees a provider at the same time every day or every week may tend to be in a particular mood at that time of day (e.g., relaxed after stopping work for the day), and may tend to rarely be in a different mood at that time of day (e.g., rarely exhausted in the late morning). Further, due to the prescheduled nature of provider sessions, a provider is limited to only being able to provide treatment during the session, as the provider may need to see other patients before or after the session and/or the individual may have other obligations before or after the session. Thus, a traditional provider is unable to time different active treatments (e.g., non-self-directed treatments) outside of the scheduled session(s) with the individual.

By contrast, certain aspects and features of the present disclosure provide for a therapy-providing system capable of providing treatment at any time, such as a time when the individual will be most receptive to the treatment, even if that time is not prescheduled. Further, the system is capable of obtaining information about the individual's mood at any time throughout the day (e.g., by proactive user action to provide user input, or in response to a prompt for user input), not merely during a pre-scheduled session or whenever the user remembers to self-track mood information outside of a pre-scheduled session.

As such, the therapy-providing system (e.g., chatbot) disclosed herein is capable of providing to a user highly personalized therapy that would otherwise be unattainable with traditional therapeutic approaches. The personalized nature of the provided therapy has several substantial benefits, such as i) providing an optimized or otherwise improved response to the provided therapy; ii) improving the user's appreciation of the provided therapy and the therapy-providing system; and iii) establishing and improving the bond of trust, or working alliance, between the user and the therapy-providing system.

The therapy-providing system can be a system of one or more computing devices that facilitate providing therapy to the user. In some cases, therapy can be provided in the form of suggesting a therapy tool with which the user can self-engage, such as suggesting the user perform known exercises or suggesting the user perform written exercises in a book. In other cases, however, providing therapy includes actively engaging the user with a therapy tool, which can include providing prompts, receiving user input, providing responsive feedback, and/or otherwise engaging the user according to the directives of the therapy tool.

A therapy tool, also known as a therapeutic, can be an exercise, technique, workflow, or other user-engageable action or set of actions designed to achieve a therapeutic response. Examples of therapy tools or classes of therapy tools include journaling, cognitive restructuring, behavioral activation, sleep optimization, SMART (specific, measurable, achievable, relevant, time-bound) goals support, attention retraining, craving intervention, distraction tool sequencing, mindfulness exercises, distress tolerance, controlled breathing, urge surfing, communication skills training, relationship troubleshooting, grief exercises, loneliness exercises, social skills building, and others.

In an example, providing the therapy tool of journaling can include i) prompting (e.g., reminding) a user to record thoughts into a journal; ii) facilitating entry of thoughts into a journal, such as by providing prompts to help the user identify what to record into the journal, or by providing prompts to which the user can respond such that the responses can be stored in the journal; or iii) automatically pre-populating journal entries based on prior user interactions.

In another example, providing the therapy tool of cognitive restructuring can include providing prompts to walk the user through steps of cognitive restructuring, such as i) identifying the situation; ii) identifying the thoughts (and/or feelings) evoked from the situation; iii) identifying evidence that supports the thoughts; iv) identifying evidence that does not support the thoughts; v) identifying alternative or more balanced thoughts; and vi) identifying the outcome from adopting the new thoughts. When the therapy-providing system is a chatbot, the chatbot can provide prompts to the user, requesting the user provide input answering the prompts. For example, the chatbot can request the user identify thoughts or feelings associated with a described situation. In some cases, the user input can be received as free text (e.g., text typed in by the user, which can be in the user's own words), although in other cases, the user input can be received as constrained text options (e.g., a user selecting one or more pre-populated responses, such as by tapping on a button or checking a checkbox).

In cases where the therapy-providing system is implemented as a chatbot, inputs are often received in the form of text selected from a list (e.g., constrained text) or entered into a field (e.g., free text), although that need not always be the case. For example, in some cases, individuals can speak or dictate to the chatbot. Likewise, while a chatbot may generally provide prompts and/or otherwise communicate to the user via text, in some cases a chatbot can use a text-to-speech engine to read out responses.

While described with reference to a chatbot in many places herein, certain aspects and features of the present disclosure can be used for other purposes, such as to provide personalized therapy in human-human interacts, such as text-based or audio-based communications between individuals locally or remotely. For example, a therapist treating a patient may make use of a system that, instead of providing personalized therapy itself, generates personalized therapy recommendations, which can be leveraged by the therapist to improve the level of personalization the therapist is able to provide to the patient. For example, a system capable of identifying a therapy sequencing that is best for a user's mood and/or recent assessment(s) can be leveraged by a therapist to deliver therapy according to the identified therapy sequencing.

Certain aspects of the present disclosure involve collecting mood information about a user via user input. User input data (e.g., free text, constrained text, and others) can be collected either at the user's own instigation (e.g., the user opens the chatbot and specifically states their mood) or in response to a prompt from the therapy-providing system (e.g., during a chatbot session, the chatbot asks the user about their mood and the user responds). The user input data can be analyzed to generate a mood score. Mood scores can be an indication of a severity or intensity of one or more moods. In some cases, an overall mood score is used. The overall mood score can represent the user's overall mood in terms of a positive or negative mood. In some cases, however, mood scores can include enumerated mood scores. Each enumerated mood score indicates the severity or intensity of a specified mood, such as "happiness," "anger," or "curiosity." Any state of mind or feeling can be represented as an enumerated mood. In some cases, however, the therapy-providing system makes use of a limited set of enumerated moods.

Enumerated moods can be presented in various fashions. In some cases, a particular enumerated mood score can be indicative of a level of that mood and a level of an opposing mood. For example, in some cases a "happiness" mood score can be representative of a level of happiness from unhappy (e.g., -100) to happy (e.g., 100). In other cases, however, each enumerated mood score can represent only that particular enumerated mood. For example, in such cases, a "happiness" mood score can be representative of a level of happiness (e.g., from 0 to 100) and a separate "unhappiness" mood score can be simultaneously representative of a level of unhappiness (e.g., from -100 to 0 or from 0 to 100). As used herein, the term "unique mood" can be inclusive of fully unique moods (e.g., "happiness" and "unhappiness" can be unique moods that represent a level of happiness and a level of unhappiness, respectively) or semi-unique moods that are indicative of both a positive and negative version of a mood (e.g., "happiness" can be a unique mood that represents both a level of happiness and a level of unhappiness).

Mood scores can be generated based on the user input data. In some cases, the user input data can be directly used to generate an overall mood score and/or one or more enumerated mood scores. In some cases, an overall mood score can be calculated based on one or more enumerated mood scores. Mood scores can have any suitable form, although in some cases mood scores can have values between a minimum score and a maximum score. In some cases, mood score value ranges can extend from a negative number up to a positive number, in which case negative numbers can indicate a generally negative mood and positive numbers can indicate a generally positive mood. For example, a happiness mood score of -34 may indicate the user is relatively unhappy, whereas a happiness mood score of 82 may indicate the user is very happy. In some cases, however, a mood score may be only positive or only negative.

Moods can be tracked on demand and/or regularly (e.g., twice daily, daily, multiple times per week, weekly, and the like). Moods can be tracked before a therapy tool is applied (e.g., at the beginning of a chatbot session before a therapy tool is used), while a therapy tool is being applied (e.g., input data indicative of a user's mood may be provided as part of a therapy tool), and/or after a therapy tool is applied (e.g., at the end of a chatbot session after a therapy tool has been used). Tracking a mood includes receiving user input data and generating a mood score (e.g., creating or adjusting one or more mood scores) based on the user input data.

In an example, a user may provide input data by selecting one of a set of available mood inputs. For example, the therapy-providing system may prompt the user to select one or more moods from the list including anxious, sad, tired, happy, angry, depressed, sick, content, really happy, indifferent, upset, distressed, relieved, neutral, shocked, angry, and curious. Other moods can be used, and in some cases a user can provide free text to indicate a mood). Upon selecting one or more moods, that user input data can be used to generate (e.g., create and/or update) a mood score. The user input data may affect a single enumerated mood score (e.g., selecting "happy" may only affect a happiness mood score, such as by increasing it) or multiple enumerated mood scores (e.g., selecting "distressed" may cause a happiness mood score to decrease slightly and an anxiety mood score to increase more).

By tracking the user's mood before and after a therapy tool is applied, and optionally during, the therapy-providing system can determine how the therapy tool affected the user's mood(s). For example, if a user has a pre-therapy happiness mood score of 31 and a post-therapy happiness mood score of 42, an assumption can be made that the therapy had a positive effect on the user's happiness.

Mood scores and related therapy tool information (e.g., identification of therapy tool used and/or other information about the application of the therapy tool) can be collected over time. In some cases, mood scores collected over time can be used to identify mood score trends. Collected mood scores (and optionally mood score trends) and related therapy tool information can be used to train a mood-based personalization model. A mood-based personalization model can be a machine learning algorithm trained to generate outputs based on input mood information, such as mood scores and/or mood score trends. The generated outputs can depend on the desired function(s) of the personalization model, as discussed in further detail below.

In some cases, the mood-based personalization model can be trained to output a therapy tool selection, which can be a selection of a particular therapy tool out of a set of available therapy tools. For example, the level of intensity of one or more mood scores may dictate which tool is best able to address the mood (e.g., a user with a anger score of 5 out of 100 may benefit most from a cognitive restructuring exercise, whereas the user may benefit most from a controlled breathing exercise if the user's anger score is 55 out of 100). Further, some therapy tools may work best when the user is experiencing certain moods. Some therapy tools may provide a more reliable, larger, faster, and/or more durable improvement depending on the mood or combination of moods being experienced by the user. In some cases, the therapy-providing system can request further input from the user regarding what type of outcome they desire, such as a more reliable improvement, a larger improvement, a faster improvement, or a more durable improvement. Such further input can be applied to the personalization model to identify the most appropriate therapy tool to be used. The mood-based personalization model can be trained using historic training data to achieve these types of outputs.

In some cases, the mood-based personalization model can be trained to output therapy sequencing, which can be a sequence, or order, of two or more therapy tools to be applied to the user. For example, one or more moods may take priority over other moods, such as if the presence of the priority mood (e.g., an enumerated mood score for the priority mood is at or above a threshold value) would interfere with treatment of other moods (e.g., strong anxiety or depression may interfere with treatment of other moods, and thus the sequencing may first use a therapy tool designed to treat the anxiety or depression before using a therapy tool designed to treat the other mood(s)). In some cases, some moods will always be deemed priority moods (e.g., suicidal thoughts), whereas other moods will sometimes be prioritized over others depending on the individual (e.g., anxiety versus depression). In some cases, the prioritization of moods depends on the intensity of the moods. For example, if one of the enumerated mood scores is especially high, it may be prioritized over other enumerated mood scores that are not as high. In some cases, prioritization of moods can depend on the likelihood of success of a treatment tool improving the mood. For example, if a particular mood rarely improves when a treatment tool is applied (e.g., across a cohort or for an individual), that particular mood may be deemed low in priority as compared to other moods, and thus be treated later in the treatment sequencing than those other moods. The mood-based personalization model can be trained using historic training data to achieve these types of outputs.

In some cases, the mood-based personalization model can be trained to output a therapy timing, which can be an indication of when (e.g., a particular time or day; or a particular frequency, such as daily or weekly) therapy is to be provided to the user. For example, certain moods may be more effectively treated if therapy is provided on a daily basis (e.g., strong anxiety or depression), whereas other moods may be more effectively treated if therapy is provided only occasionally, such as weekly (e.g., tired). The mood-based personalization model can be trained using historic training data to achieve these types of outputs.

Training of a mood-based personalization model can be individual-based and/or cohort-based. Individual-based training involves training the model using historical data associated with the individual, such as the individuals past mood scores and past applied therapy tools. Such a model would be useful to provide highly personalized therapy to the user, but may require many data points. Cohort-based training involves training the model using historical data associated with multiple individuals in a cohort. The multiple individuals in the cohort may share one or more common features, such as gender, age, occupation, symptoms, diagnoses, or any other discernable feature. Once trained, the cohort-trained mood-based personalization model can be used with existing and new users (e.g., new users in the same cohort), alike. Thus, new users without available historical data can still benefit from the pre-trained cohort-trained mood-based personalization model and still receive personalized therapy. In some cases, a cohort-trained model is initially used, then further updated using individual-based training. In some cases, a single cohort-trained model can be used for all users of the therapy-providing system, although that need not always be the case. In some cases, the therapy-providing system can access a plurality of cohort-trained models and select a single cohort-trained model that fits an identified cohort of the user.

In addition to or instead of moods and mood scores, the therapy-providing system can track user-provided assessments of previously applied therapy and/or associated therapy targets. As used herein, the term therapy target is inclusive of problems or conditions that therapy is intended to treat. Examples of therapy targets include depression, difficulty falling asleep, uncontrollable anger, posttraumatic stress disorder, eating disorders, paranoia, and the like. Therapy targets can range from very broad (e.g., depression), to moderately broad (e.g., postpartum depression), to narrow (e.g., restlessness, anxiety, and irritability). As such, some therapy targets can be broken down into other discrete therapy targets.

Assessments can be collected via user input. User input data can be collected either at the user's own instigation (e.g., the user opens the chatbot and specifically states that they are having difficulty sleeping) or in response to a prompt from the therapy-providing system (e.g., during a chatbot session, the chatbot asks the user to rank how often they fall asleep when they want to and the user responds). In some cases, user input data associated with assessments can include selecting from a list of available responses. For example, a therapy-providing system may collect assessment data by asking the user to answer a series of questions, each to which the user can respond by selecting from a list of available responses (e.g., "All of the time (3), Most of the time (2), Some of the time (1), None of the time (0)").

The user input data can be analyzed to generate one or more assessment scores. Assessment scores can be an indication of a severity or intensity of one or more therapy targets. In some cases, an overall assessment score is used. The overall assessment score can represent the user's overall severity of all therapy targets associated with the user. In some cases, however, assessment scores can include category assessment scores. Each category assessment score indicates the severity or intensity of a specified therapy target or aspect of a therapy target. Any addressable problem or condition can be represented as a category assessment score. In some cases, however, the therapy-providing system makes use of a limited set of category assessment scores.

Assessment scores can be generated based on the user input data. In some cases, the user input data can be directly used to generate an overall assessment score and/or one or more category assessment scores. In some cases, an overall assessment score can be calculated based on one or more category assessment scores. Assessment scores can have any suitable form, although in some cases assessment scores can have values between a minimum score and a maximum score. In some cases, assessment score value ranges can extend from a negative number up to a positive number, in which case negative numbers can indicate a generally negative assessment and positive numbers can indicate a generally positive assessment. In some cases, however, an assessment score may be only positive or only negative. In some cases, a higher assessment score is indicative of a worse problem, although that need not always be the case.

Assessment scores can be tracked on demand and/or regularly (e.g., twice daily, daily, multiple times per week, weekly, and the like). Assessment scores can be tracked before a therapy tool is applied (e.g., at the beginning of a chatbot session before a therapy tool is used), while a therapy tool is being applied (e.g., input data indicative of a user's mood may be provided as part of a therapy tool), and/or after a therapy tool is applied (e.g., at the end of a chatbot session after a therapy tool has been used). Tracking an assessment score includes receiving user input data and generating an assessment score (e.g., creating or adjusting one or more assessment scores) based on the user input data.

In an example, a user may provide input data in response to an assessment survey associated with a particular therapy target. For example, the therapy-providing system may prompt the user to identify "How often did you fall asleep when you wanted to in the past week? All of the time (3), Most of the time (2), Some of the time (1), or None of the time (0)" to which the user may respond with an appropriate selection (e.g., 2 - most of the time). Upon providing the response, and any additional responses as needed, that user input data can be used to generate (e.g., create and/or update) an assessment score. The user input data may affect a single category assessment score (e.g., in an example, selecting "Most of the time (2)" may only affect a category assessment score associated restlessness) or multiple category assessment scores (e.g., in another example, selecting "Most of the time (2)" may affect a category assessment score associated restlessness and a separate category assessment score associated with postpartum depression).

By tracking the user's assessment score(s) before and after a therapy tool is applied, and optionally during, the therapy-providing system can determine how the therapy tool affected the user's assessment of one or more therapy targets. For example, if a user has a pre-therapy category assessment score for depression of 31 and a post-therapy category assessment score for depression of 20, an assumption can be made that the therapy had a beneficial effect by reducing the user's depression.

Assessment scores and related therapy tool information (e.g., identification of therapy tool used and/or other information about the application of the therapy tool) can be collected over time. In some cases, assessment scores collected over time can be used to identify assessment score trends. Collected assessment scores (and optionally assessment score trends) and related therapy tool information can be used to train an assessment-based personalization model. An assessment-based personalization model can be a machine learning algorithm trained to generate outputs based on input assessment data, such as assessment scores and/or assessment score trends. The generated outputs can depend on the desired function(s) of the personalization model, as discussed in further detail below.

In some cases, the assessment-based personalization model can be trained to output a therapy tool selection, which can be a selection of a particular therapy tool out of a set of available therapy tools. For example, the level of intensity of one or more assessment scores may dictate which tool is best able to address the therapy target (e.g., a user with a category assessment score for depression of 5 out of 100 may benefit most from a first type of therapy tool, whereas the user may benefit most from a second type of therapy tool if the user's category assessment score for depression is 55 out of 100). Further, some therapy tools may work best when the user is showing a particular assessment score trend. For example, a first therapy tool may have low effectivity when the user is just starting to show improvements in a particular assessment score, and thus a different tool may be used, but when the user starts showing stronger improvements in that assessment score, the therapy-providing system may instead provide the first therapy tool. Some therapy tools may provide a more reliable, larger, faster, and/or more durable improvement in assessment score depending on the current assessment score, combination of assessment scores, and/or assessment score trend(s) associated with the therapy targets of the user. In some cases, the therapy-providing system can request further input from the user regarding what type of outcome they desire, such as a more reliable improvement, a larger improvement, a faster improvement, or a more durable improvement. Such further input can be applied to the personalization model to identify the most appropriate therapy tool to be used. The assessment-based personalization model can be trained using historic training data to achieve these types of outputs.

In some cases, the assessment-based personalization model can be trained to output therapy sequencing, which can be a sequence, or order, of two or more therapy tools to be applied to the user. For example, certain therapy targets may take priority over other moods, such as if the presence of a priority therapy target (e.g., a category assessment score for the priority therapy target is at or above a threshold value) would interfere with treatment of other therapy targets (e.g., strong anxiety or depression may interfere with treatment of therapy targets, and thus the sequencing may first use a therapy tool designed to treat the anxiety or depression before using a therapy tool designed to treat the other therapy targets). In some cases, some therapy targets will always be deemed priority therapy targets (e.g., suicidal thoughts), whereas other therapy targets will sometimes be prioritized over others depending on that individual's personal assessment scores and/or assessment trends. In some cases, the prioritization of therapy targets depends on the intensity of the therapy target (e.g., the value of the category assessment score for that therapy target). For example, if one of the category assessment scores is especially high, its therapy target may be prioritized over those of other category assessment scores that are not as high. In some cases, prioritization of therapy targets can depend on the likelihood of success of a treatment tool improving an assessment score (e.g., the overall assessment score, a category assessment score for that therapy target, and/or category assessment scores for one or more other therapy targets). For example, if a particular therapy target rarely improves when a treatment tool is applied (e.g., across a cohort or for that particular individual), that particular therapy target may be deemed low in priority as compared to other therapy targets, and thus be treated later in the treatment sequencing than those other therapy targets. The assessment-based personalization model can be trained using historic training data to achieve these types of outputs.

In some cases, the assessment-based personalization model can be trained to output a therapy timing, which can be an indication of when (e.g., a particular time or day; or a particular frequency, such as daily or weekly) therapy is to be provided to the user. For example, certain therapy targets may be more effectively treated if therapy is provided on a daily basis (e.g., strong anxiety or depression), whereas other therapy targets may be more effectively treated if therapy is provided only occasionally, such as weekly (e.g., difficulty sleeping). In some cases, the timing of treating a therapy target can depend on one or more assessment scores and/or one or more assessment score trends. For example, if it appears that the assessment score for a particular therapy target is decreasing, the therapy-providing system may increase the frequency for providing therapy to that therapy target. On the other hand, if it appears that the assessment score for that therapy target is increasing, the therapy-providing system may decrease the frequency for providing therapy to that therapy target. The assessment-based personalization model can be trained using historic training data to achieve these types of outputs.

Training of an assessment-based personalization model can be individual-based and/or cohort-based. Individual-based training involves training the model using historical data associated with the individual, such as the individuals past assessment scores and past applied therapy tools. Such a model would be useful to provide highly personalized therapy to the user, but may require many data points. Cohort-based training involves training the model using historical data associated with multiple individuals in a cohort. The multiple individuals in the cohort may share one or more common features, such as gender, age, occupation, symptoms, diagnoses, or any other discernable feature. Once trained, the cohort-trained assessment-based personalization model can be used with existing and new users (e.g., new users in the same cohort), alike. Thus, new users without available historical data can still benefit from the pre-trained cohort-trained assessment-based personalization model and still receive personalized therapy. In some cases, a cohort-trained model is initially used, then further updated using individual-based training. In some cases, a single cohort-trained model can be used for all users of the therapy-providing system, although that need not always be the case. In some cases, the therapy-providing system can access a plurality of cohort-trained models and select a single cohort-trained model that fits an identified cohort of the user.

In some cases, a combination of an assessment-based personalization model and mood-based personalization model can be used. In other words, in some cases, a mood-based personalization model can be trained with training data containing assessment score information or an assessment-based personalization model can be trained with training data containing mood score information, thus resulting in a personalization model that takes both mood scores and assessment scores into account.

Generally, a personalization model is trained using machine learning techniques, such as supervised learning or unsupervised learning. In some cases, however, a personalization model can be programmed using non-machine-learning techniques. Such a non-machine-learning personalization model can provide some of the benefits described herein by using predefined rules and logic. For example, a non-machine-learning assessment-based personalization model may control the frequency with which the therapy-providing system provides a particular therapy (e.g., prompting the user to engage in a therapy tool) based on one or more recent assessment scores. In some cases, non-machine-learning personalization models may be unable to provide as effective therapy as machine-learning personalization models.

Certain aspects and features of the present disclosure include the collection of mood information and/or assessment information over the course of time for one or more individuals. In such cases, it collection of mood information and/or assessment information can include establishing a timestamp associated with the collection of the information. For example, when user input is received to generate a mood score, a timestamp can be created in association with that mood score, with the timestamp indicating the time when the user input is provided. As another example, when user input is received to generate an assessment score, a timestamp can be created in association with that assessment score, with the timestamp indicating the time when the user input is provided. Additionally, timestamps can be created whenever therapy is provided to the user. For example, when therapy is provided to a user via a chatbot, a timestamp can be created, and optionally stored with other therapy-related information, indicating when the therapy was provided (e.g., a time when therapy was initially provided, a time when therapy was completed, or another suitable time associated with providing the therapy). By generating the various timestamp data described herein, the therapy-providing system is able to compare scores (e.g., mood scores and/or assessment scores) over time, as well as compare how scores change with respect to provided therapy. For example, efficacy of a particular therapy can be inferred from a change in assessment score when a first assessment score with a first timestamp is compared to a second assessment score with a second timestamp, when a therapy timestamp for the provided therapy falls between the first timestamp and the second timestamp. Additionally, by automating the process of timestamp generation in association with scores and/or provided therapy, personalization model(s) can be trained with high accuracy to facilitate delivery of improved, future therapy.

Aspects and features of the present disclosure are associated with the practical application of automatically providing therapy to a user that is dynamically personalized to that user. Certain hardware and software implementations of certain aspects and features of the present disclosure are used to provide particular treatment and/or prophylaxis for mental health disorders. The personalized nature of the provided therapy permits the treatment and/or prophylactic effect to be even stronger than otherwise obtainable in a solely human-to-human interaction.

Aspects and features of the present disclosure provide various improvements to the technological process human-computer interaction, especially with respect to chatbots, such as therapy chatbots. Examples of such improvements include i) an ability to better track a user's mood and its effects on different therapeutic approaches, ii) an ability to better track a user's mental health assessments and their effects on different therapeutic approaches, and iii) an ability to leverage i and ii to provide improved therapy via the chatbot.

These illustrative examples are given to introduce the reader to the general subject matter discussed here and are not intended to limit the scope of the disclosed concepts. The following sections describe various additional features and examples with reference to the drawings in which like numerals indicate like elements, and directional descriptions are used to describe the illustrative embodiments but, like the illustrative embodiments, should not be used to limit the present disclosure. The elements included in the illustrations herein may not be drawn to scale.

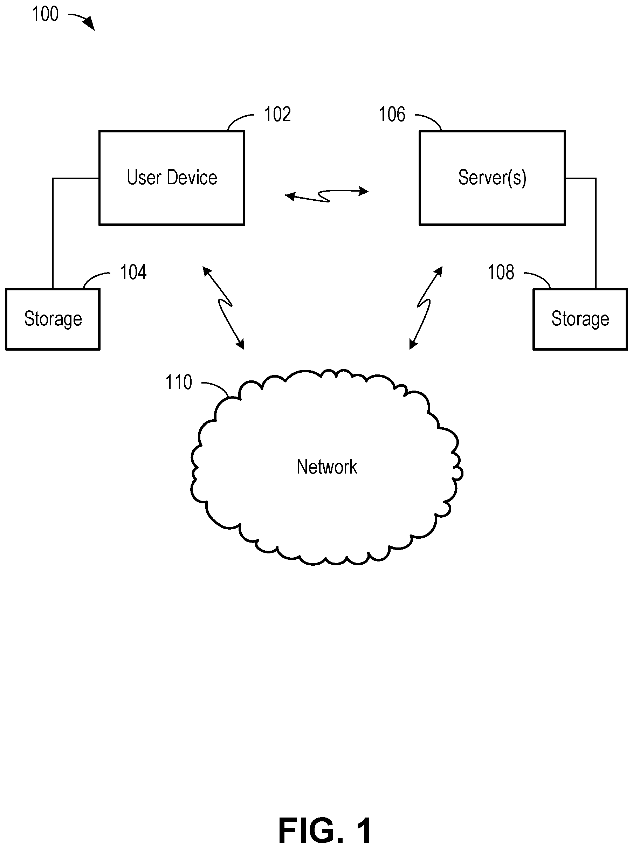

FIG. 1 is a schematic diagram depicting a computing environment 100 according to certain aspects of the present disclosure. The environment 100 can be located in a single physical location or can be distributed about multiple physical locations. Environment 100 can be used to implement a therapy-providing system, such as a chatbot or automated therapy system, such as one being used to receive user input, process the user input to generate one or more mood scores and/or one or more assessment scores, then provide personalized therapy based on these scores, such as described in further detail herein. Environment 100 is an example of a suitable environment for implementing the system, although other environments can be used instead.

Environment 100 can include a user device 102 and a server 106, although in some cases one of these devices may not be included. For example, some environments contain only a user device 102. In some cases, multiple user devices 102 can be used. In some cases, other devices can be used. When multiple devices are used, each device can be communicatively coupled together, such as via a direct connection (e.g., a wired connection, such as a universal serial bus (USB) connection, or a wireless connection, such as a Bluetooth connection) or via network 110. Network 110 can be any suitable network, such as a local area network, a wide area network, a cloud, or the Internet. The system can be implemented on a single device (e.g., on user device 102) or can be implemented across multiple devices (e.g., any combination of user device 102 and sever(s) 106).

An individual can interact with the chatbot via the user device 102 and/or another device. Interacting with the chatbot can include i) establishing a chatbot session; ii) receiving prompts or notifications from the chatbot, which can optionally occur outside of an active chatbot session; iii) providing user input, such as free text, constrained text, selections, and the like; iv) receiving therapy, such as in the form of a therapy tool applied by the chatbot as one or more text entries, including statements or questions; or v) any combination of i-iv.

In some cases, user input can be processed using natural language processing (NLP) techniques to attribute meaning to the provided input. For example, free text user input containing the phrase "This has been a pretty awesome day so far!" may be interpreted by NLP techniques to represent an indication that a particular enumerated mood score or category assessment score should be adjusted in a positive direction.

A user device 102 can act as a primary mode of interaction for one or more individuals to provide user input; receive responses, prompts, and information; and otherwise interact with the system. Examples of user device 102 include any suitable computing device, such as a personal computer, a smartphone, a tablet computer, a smartwatch, a computerized audio recorder, or the like. User device 102 can be operatively coupled to storage 104 to store data associated with applications and processes running on the user device 102. User device 102 can include any combination of input/output (I/O) devices that may be suitable for interacting with the system, such as a keyboard, a mouse, a display, a touchscreen, a microphone, a speaker, an inertial measurement unit (IMU), a haptic feedback device, or other such devices.

One or more servers 106 can be used to enable processes and techniques disclosed herein, such as generating scores (e.g., mood scores and/or assessment scores), training personalization models, selecting therapy tools, determining therapy sequencing, determining therapy timing, and applying personalized therapy (e.g., in the form of text prompts and responses transmitted via the chatbot). The server(s) 106 can receive user input from user device 102 via network 110, and can provide output (e.g., chatbot output) by transmitting text or other output to the user device 102 via network 110.

Mood score(s), assessment score(s), provided therapy information (e.g., information about when therapy was provided, what therapy tool was used, the therapy target, and/or any other information about how the therapy was provided), and personalization models can be stored "locally" on a user's user device 102 (e.g., in storage 104) and/or "remotely" on the server(s) 106 (e.g., on storage 108). In some cases, cohort-based personalization models are stored in storage 108 of server(s) 106, whereas individual-based personalization models are stored in storage 104 of user device 102. In some cases, all personalization models are stored in storage 108 of server(s) 106.

In some cases, various processes and techniques disclosed herein can occur directly on the user device 102, such as generating an assessment score from user input data. In such cases, the user device 102 can provide outputs to the server(s) 106 other than just user input data (e.g., user input data and the generated assessment score). However, in many cases, the user device 102 is used to generate a chatbot session with the server(s) 106, transmit user input to the server(s) 106 in response to the user providing the input via an input device (e.g., keyboard or touchscreen), and present chatbot outputs (e.g., text, images, sounds, or other discernable outputs presented, such as via an output device like a screen, a speaker, a light, or the like) in response to receiving the chatbot outputs from the server(s) 106.

In some cases, environment 100 can include additional or fewer components than those depicted.

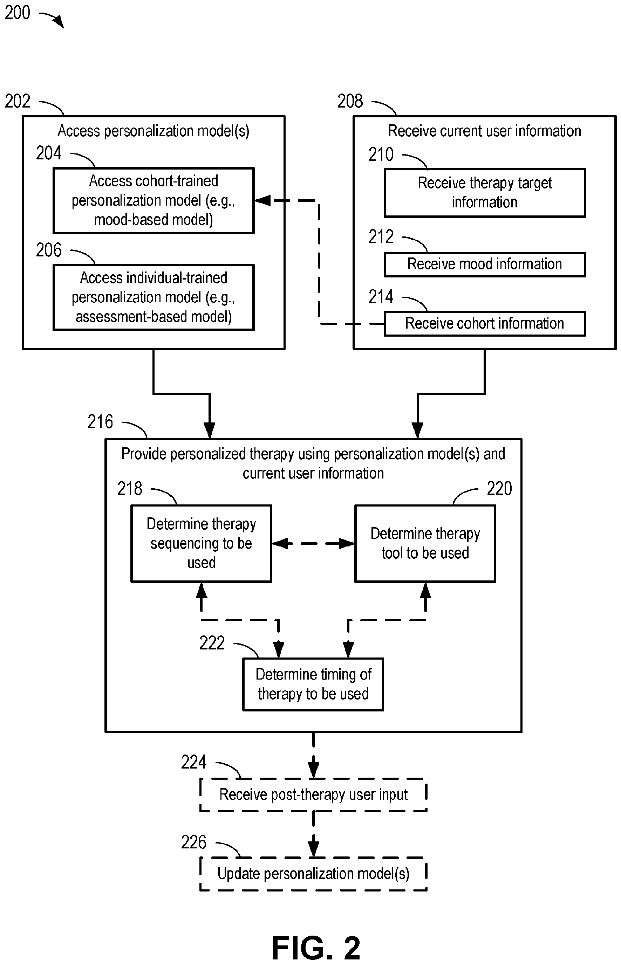

FIG. 2 is a flowchart depicting a process 200 for providing personalized therapy, according to certain aspects of the present disclosure. Process 200 can be performed by any suitable computing device or computing devices, such as server(s) 106 of FIG. 1.

At block 202, one or more personalization models can be accessed. Accessing a personalization model can include accessing a model that is stored in memory, such as memory accessible to a server running a therapy chatbot. Accessing one or more personalization models can include accessing a cohort-trained personalization model at block 204; accessing an individual-trained personalization model at block 206, or accessing both a cohort-trained personalization model at block 204 and an individual-trained personalization model at block 206. In some cases, performing one or both of blocks 204 and 206 can include accessing a single personalization model that is both cohort-trained and individual-trained (e.g., a personalization model that was originally cohort-trained, but then further trained using training data associated with a particular individual). In some cases, the cohort-trained personalization model is a mood-based personalization model, although that need not always be the case. In some cases, the individual-trained personalization model is an assessment-based personalization model, although that need not always be the case. The personalization model(s) accessed at block 202 can be used in providing personalized therapy at block 216.

At block 208, current user information can be received. Current user information can be user information associated with a current state of the user, such as a current mood, a current therapy target, a current assessment score associated with a therapy target, a current mood score trend (e.g., a number of previous scores for a particular mood score), a current assessment score trend (e.g., a number of previous scores for a particular assessment score), or any combination thereof. In some cases, receiving the current user information at block 208 can include receiving some or all of the information from the user via a chatbot interface, such as via a user device. In some cases, receiving the current user information at block 208 can include receiving some or all of the information from storage (e.g., storage 108 of server(s) 106 of FIG. 1). In some cases, receiving a piece of current user information can include generating the piece of current user information from other received user information. In an example, a current mood score trend may be generated based on a current mood score received at that time from the user via the chatbot interface and a set of historical scores for that same mood score accessed from storage (e.g., using a unique identifier associated with the user).

Receiving current user information at block 208 can include i) receiving therapy target information at block 210; ii) receiving mood information at block 212; iii) receiving cohort information at block 214; or iv) any combination of i - iii.

In some cases, receiving the current user information at block 208 can include receiving therapy target information at block 210. Receiving therapy target information can include receiving an indication of a desired therapy target from the user (e.g., via user input that says "I want to work on feeling too anxious" or user input that selects "anxiety" from a constrained list of therapy targets). In some cases, however, receiving therapy target information includes accessing (e.g., looking up) existing therapy target information associated with a user (e.g., via a unique user identifier). For example, receiving therapy target information at block 210 can include receiving a list of one or more therapy targets that the user has previously identified as therapy targets and/or that the system suspects are suitable therapy targets for the user. In some cases, receiving therapy target information at block 210 include receiving current and/or historical assessment score information associated with the therapy target.

In some cases, receiving the current user information at block 208 can include receiving mood information at block 212. Receiving mood information can include i) receiving user input data usable to generate a mood score; ii) generating a mood score from user input data; iii) accessing stored, historical user input data; iv) accessing stored, historical mood scores; or v) any combination of i-iv. In some cases, user input data usable to generate a mood score can take the form of i) directly identifying a particular mood; ii) directly identifying a severity of a particular mood; iii) providing information that suggests a particular mood; iv) providing information that suggests a severity of a particular mood; or v) any combination of i-iv.

In some cases, receiving the current user information at block 208 can include receiving cohort information at block 214. Receiving cohort information can include i) receiving a cohort identifier from the user (e.g., as a selection or an instruction); ii) accessing a stored cohort identifier based on a user identifier; iii) selecting a cohort for the user based on provided user input data (e.g., gender, age, etc.); or iv) any combination of i-iii. The cohort information can be indicative of a particular cohort of individuals. In some optional cases, the cohort information received at block 214 can be used at block 204 to access the appropriate cohort-trained personalization model, such as to select the appropriate model out of a set of possible models based on the cohort identified by the cohort information at block 214.

At block 216, personalized therapy can be provided, such as by transmitting chatbot output (e.g., text, images, etc.) for presentation on the user's user device in accordance with the personalized therapy. The therapy provided can be personalized based on the one or more personalization models accessed at block 202 and the current user information from block 208. For example, some or all of the current user information can be applied to one or more personalization models to generate model output information, which can dictate how to personalize the therapy, as described in further detail herein. In an example, cohort information received at block 214 can be used to select a particular cohort-trained personalization model at block 204, to which mood information received at block 212 can be applied to generate an output that indicates a particular therapy tool should be used to provide the personalized therapy at block 216.

Providing personalized therapy at block 216 can include i) determining a therapy sequencing to be used at block 218; ii) determining a therapy tool to be used at block 220; iii) determining a timing of therapy to be used at block 222; or iv) any combination of i-iii. In some cases, the output of any one of blocks 218, 220, 222 can be further used in the performance of one or both of the others of blocks 218, 220, 222. For example, in some cases, determining a therapy sequencing at block 218 can depend on the therapy tool(s) selected at block 220.

In some cases, a separate personalization model can be used for each of blocks 218, 220, 222, although that need not always be the case. For example, separate personalization models for blocks 218, 220, and 222 can be individually trained using training data that includes therapy sequencing, therapy tools, and therapy timing, respectively, as desired outputs.

In some cases, providing personalized therapy at block 216 can include determining a therapy sequence to be used at block 218. Determining the therapy sequence to be used can include identifying an order of one or more therapy tools to apply and/or one or more therapy targets to pursue. For example, given the current user information from block 208, a therapy sequence may be determined that calls for application of a therapy tool B before application of a therapy tool A. In another example, given the current user information from block 208, a therapy sequence may be determined that calls for treatment of a first therapy target prior to treatment of a second therapy target.

In some cases, determining the therapy sequence at block 218 includes identifying one or more "priority" therapy targets out of a set of potential therapy targets using the current user information (e.g., one or more mood scores), then ensuring the one or more "priority" therapy targets is addressed before others from the set of potential therapy targets. The priority therapy target(s) can be identified by the trained personalization model(s) and/or using predefined rules (e.g., certain therapy targets are always top priority). Like priority therapy targets, low-priority therapy targets can be identified and then addressed after others from the set of potential therapy targets. In some cases, determining the priority of a therapy target can include generating a priority score associated with each of the therapy targets (e.g., using the personalization model(s)), then ranking the therapy targets based on their individual priority scores. Other techniques can be used. In some cases, the therapy sequencing is based at least in part on the intensity of one or more scores (e.g., mood scores and/or assessment scores). For example, the therapy sequencing can emphasize one or more therapy targets based on the intensity of one or more mood scores associated with the one or more therapy targets.

When a therapy sequencing has been determined at block 218, providing personalized therapy at block 216 can include providing multiple therapy tools (e.g., presenting respective prompts to begin therapy using a respective therapy tool) in the order dictated by the therapy sequencing. In some cases, the therapy sequencing can be suggested (e.g., if a user refuses, skips, or otherwise fails to complete a therapy tool in the order provided by the therapy sequencing, the therapy-providing system can simply move to the next therapy tool in the order provided by the therapy sequencing) or mandatory (e.g., if a user refuses, skips, or otherwise fails to complete a therapy tool in the order provided by the therapy sequencing, the therapy-providing system will prohibit moving to the next therapy tool in the order provided by the therapy sequencing until the first therapy tool has been completed). In some cases, therapy sequencing can be automatic (e.g., a subsequent therapy tool can be automatically provided, such as by issuing a prompt offering to begin that subsequent therapy tool, in response to completion of a prior therapy tool) or on-demand (e.g., after completion of a first therapy tool in the order provided by the therapy sequencing, the next therapy tool will not be provided until after the user issues a subsequent request to engage in therapy).

The best therapy sequence to use can be generated natively by the personalization model(s) or can be determined via an effectiveness score output by the personalization model(s). The effectiveness score can represent a usefulness of the therapy sequencing based on the current user information from block 208. For example, given the user's current mood, the personalization model may output that applying a particular sequence of therapy tools would have an effectiveness score of 65 out of 100, and applying an alternate sequence (e.g., of the same therapy tools, of partially overlapping therapy tools, or of entirely different therapy tools) would have an effectiveness score of 86 out of 100, in which case the alternate therapy sequencing may be selected. In some cases, the effectiveness score can be based on additional criteria, such as what type of improvement is desired (e.g., a faster improvement, a more durable improvement, a larger improvement, a more reliable improvement, or the like). The additional criteria can be automatically determined, such as determined by the personalization model or already integrated into the personalization model, or can be manually provided (e.g., a user can select that they want the best tool for achieving the most reliable improvement). The therapy sequencing selected can be selected based on the ranking of the effectiveness score for each therapy sequencing. Other techniques can be used.

In some cases, providing personalized therapy at block 216 can include determining a therapy tool to be used at block 220. Determining the therapy tool to be used can include selecting one or more therapy tools out of a set of possible therapy tools. The set of possible therapy tools can be a set of all available therapy tools, or can be a set of therapy tools already associated with a given set of one or more therapy targets (e.g., the therapy target(s) received at block 210). For example, given the current user information from block 208, the therapy-providing system may determine that it would be more beneficial (e.g., result in faster improvement, a more durable improvement, a larger improvement, a more reliable improvement, or the like) to use a therapy tool B instead of therapy tool A.

The best therapy tool to use can be generated natively by the personalization model(s) or can be determined via an effectiveness score output by the personalization model(s). In some cases, selecting a therapy tool to use can include generating a therapy tool effectiveness score for each of the possible therapy tools. The effectiveness score can represent a usefulness of the therapy tool based on the current user information from block 208. For example, given the user's current mood, the personalization model may output that therapy tool A would have an effectiveness score of 65 out of 100 and therapy tool B would have an effectiveness score of 86 out of 100, in which case therapy tool B may be selected. In some cases, the effectiveness score can be based on additional criteria, such as what type of improvement is desired (e.g., a faster improvement, a more durable improvement, a larger improvement, a more reliable improvement, or the like). The additional criteria can be automatically determined, such as determined by the personalization model or already integrated into the personalization model, or can be manually provided (e.g., a user can select that they want the best tool for achieving the most reliable improvement). The therapy tool(s) selected can be selected based on the ranking of each therapy tool's effectiveness score. Other techniques can be used. In some cases, the selection of which therapy tool to use is based at least in part on the intensity of one or more scores (e.g., mood scores and/or assessment scores).

In some cases, providing personalized therapy at block 216 can include determining a therapy timing to be used at block 222. Determining the therapy timing to be used can include determining i) a frequency for applying one or more therapy tools; ii) a future time to apply the one or more therapy tools; iii) a frequency for addressing one or more therapy targets; iv) a future time for addressing one or more therapy targets; or v) a combination of i - iv. For example, given the current user information from block 208, the therapy-providing system may determine that it would be more beneficial (e.g., result in faster improvement, a more durable improvement, a larger improvement, a more reliable improvement, or the like) to repeat application of a given therapy tool and/or therapy sequencing daily instead of weekly, or in the morning instead of at night.

The best frequency and/or time to address therapy target(s) and/or apply therapy tool(s) can be generated natively by the personalization model(s) or can be determined via an effectiveness score output by the personalization model(s). The effectiveness score can represent a usefulness of the therapy timing based on the current user information from block 208. For example, given the user's current mood, the personalization model may output that applying a particular therapy tool daily would have an effectiveness score of 65 out of 100, and applying the particular therapy tool weekly would have an effectiveness score of 86 out of 100, in which case the therapy timing of weekly application of the particular therapy tool may be selected. In some cases, the effectiveness score can be based on additional criteria, such as what type of improvement is desired (e.g., a faster improvement, a more durable improvement, a larger improvement, a more reliable improvement, or the like). The additional criteria can be automatically determined, such as determined by the personalization model or already integrated into the personalization model, or can be manually provided (e.g., a user can select that they want the best tool for achieving the most reliable improvement). The therapy tool(s) selected can be selected based on the ranking of each therapy timing's effectiveness score. Other techniques can be used. In some cases, the therapy timing is based at least in part on the intensity of one or more scores (e.g., mood scores and/or assessment scores).

When a therapy timing has been determined at block 222, providing personalized therapy at block 216 can include providing one or more therapy tools at times dictated by the determined therapy timing. In some cases, presenting therapy tools at times dictated by the determined therapy timing can include presenting a prompt or notification to the user to engage in therapy. In some cases, this prompt or notification can be presented with or without an active chatbot session. In an example, presenting such a notification can include determining the time for the notification and storing the notification with the time into local storage of the user's device, permitting the user's device to automatically present the notification (e.g., as an alert) when the time is reached. In another example, presenting such a notification can include determining the time for the notification and pushing a notification (e.g., as an alert) to the user's device when the time is reached.

At optional block 224, post-therapy user input can be received. Receiving post-therapy user input can include receiving user input data indicative of the user's mood and/or an assessment of a therapy target after completion of the therapy. This post-therapy user input can be used at block 226 to further update the personalization model(s), such as to further update the individual-trained personalization model from block 206.

Process 200 is depicted with certain blocks in certain orders, although that need not always be the case. In some cases, process 200 can include fewer, additional, or different blocks, and in different orders.

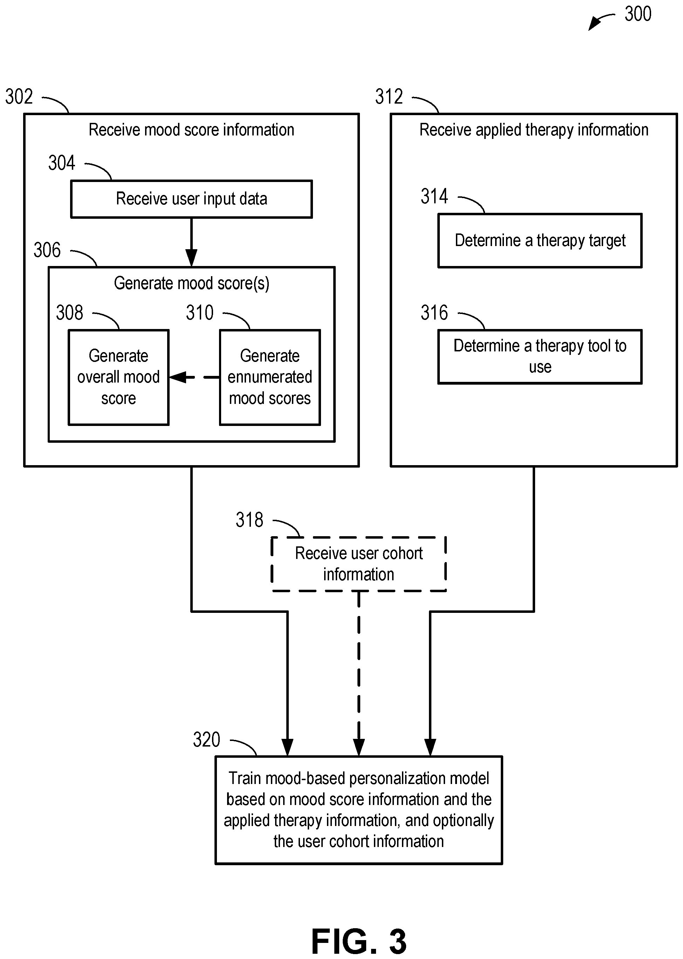

FIG. 3 is a flowchart depicting a process 300 for training a mood-based personalization model, according to certain aspects of the present disclosure. Process 300 can be performed by any suitable computing device or computing devices, such as server(s) 106 of FIG. 1. Mood-based personalization model can be any suitable model, such as the cohort-trained personalization model from block 204 of FIG. 2.

At block 302, mood score information can be received. In some cases, receiving mood score information includes accessing one or more existing mood scores, such as one or more mood scores provided by a user device. In some cases, however, receiving mood score information includes receiving user input data at block 304 and generating one or more mood scores at block 306. Generating the mood score(s) at block 306 can include using the user input data to create and/or update one or more mood scores. In some cases, the user input data can be a numerical value that is the mood score, such as if the user is prompted to score their own mood(s). In other cases, however, the user input data is analyzed to determine how to generate the mood score(s), such as by determining whether the user input data indicates a particular mood score has improved or declined since a prior measurement.

In some cases, generating the mood score(s) at block 306 can include generating an overall mood score at block 308 and/or generating one or more enumerated mood scores at block 310. An overall mood score is indicative of the user's overall mood state, and not any one specific mood. Each enumerated mood score, on the other hand, is indicative of the intensity or severity of a specific mood (e.g., happy, sad, curious, anxious, etc.).

An overall mood score can be generated at block 308 i) based directly on the user input data; ii) based on one or more enumerated mood scores generated at block 310; or iii) based on a combination of i and ii. In an example of direct generation of the overall mood score, in response to a question asking "how are you doing today, on a scale of 1 to 10?" the user may say "7," in which case the overall mood score may be set to 7 out of 10, 70 out of 700, or some other comparable value. In an example of generating a mood score based on one or more enumerated mood scores, the system may generate three enumerated mood scores, as described in further detail herein, with the values of 37, 42, and 89, all out of 100. In such an example, the overall mood score may be an average of the values, or 56 out of 100.

At block 310, one or more enumerated mood scores can be generated. An enumerated mood score is considered "enumerated" because it is associated with a particular named mood. In some cases, a single enumerated mood score can be further broken down into multiple additional enumerated mood scores. Generating an enumerated mood score at block 310 can include analyzing the user input data from block 304 to calculate and/or update an enumerated mood score. For example, in response to a question asking "how anxious are you feeling today, on a scale of 1 to 10?" the user may say "4," in which case the enumerated mood score for anxiety (e.g., an anxiety mood score or an anxiety score) can be set at 4 out of 10, 40 out of 100, or some other comparable value. In another example, the system may prompt the user to select one or more moods that they are currently experiencing. Based on the selection, the system may update the enumerated mood score(s) associated with the selected mood(s), as well as other enumerated mood score(s) that have a relationship to the selected mood(s). For example, if the user says they are "Happy" and "Curious," the system may increment a happy mood score and a curious mood score, decrement a sad mood score, decrement a depressed mood score, and increment a playful mood score.

Receiving mood score information at block 302 can include receiving mood score information collected over the course of an extended period of time, such as days, weeks, months, or years. In some cases, receiving mood score information at block 302 includes receiving mood score information for a single individual (e.g., to train an individually-trained model) and/or a corpus of individuals (e.g., to train a corpus-trained model). Mood score(s) can be associated with timestamps or other timing information. The timestamps or other timing in formation can be compared with applied therapy information from block 312 to determine which mood scores are pre-therapy and post-therapy for a given application of therapy. In some cases, mood score(s) can be labeled as pre-therapy and/or post-therapy with respect to a given application of therapy.

At block 312, applied therapy information can be obtained. Applied therapy information includes information about therapies that have been previously applied to the individual or to the corpus of individuals. The applied therapy information can include information such as the therapy tool used, the therapy target that was targeted, timestamps or other timing information, and the like.

In some cases, receiving applied therapy information at block 312 can include determining a therapy target at block 314. Determining a therapy target can include receiving an indication of a therapy target from a user and/or accessing a list of therapy targets associated with the user. In some cases, receiving applied therapy information at block 312 can include determining a therapy tool to use at block 316. Determining a therapy tool to use at block 316 can include selecting, automatically or from user input, a therapy tool out of a set of possible therapy tools. The determined therapy target and/or selected therapy tool can be used as applied therapy information, such as when timestamped based on when therapy was applied using the selected therapy tool and/or for the determined therapy target.

In some optional cases, user cohort information can be received at block 318. Receiving user cohort information can include receiving information about the individual and/or the individuals within the cohort of individuals, which can be used to assign the individual(s) to a cohort. A unique cohort identifier can be assigned to all individuals that make up that cohort.

At block 320, a mood-based personalization model can be trained. The mood-based personalization model can be trained based at least in part on the received mood score information from block 302, the received applied therapy information from block 312, and optionally, the received user cohort information from block 318.

Depending on whether mood score information and applied therapy information is received from an individual or a corpus of individuals, training the mood-based personalization model can result in either an individual-trained model or a corpus-trained model. In some cases, training a model at block 320 can include further training an existing model.

Training a model at block 320 can include using the received mood score information and received applied therapy information, collectively the training data, to generate desired outputs. The desired outputs can be set depending on the needs of the model. Desired outputs include those discussed with reference to block 216 of FIG. 2. For example, a mood-based personalization model can be trained to identify the optimal therapy tool to use for a given set of mood scores. In an example, training the personalization model can include setting a goal of achieving the largest improvement in a depression mood score, which can be determined from sequential mood score information. As the training data is processed, the model can be adjusted until is able to output, for any given set of inputs (e.g., a set of current mood scores for a user), the proper therapy tool expected to achieve the largest improvement in the user's depression mood score. For example, if the training data shows that users with a mood score around 30 tend to show the largest improvement with therapy tool A and users with a mood score around 70 tend to show the largest improvement with a therapy tool B, the output from the trained personalization model should indicate therapy tool A for users with a depression mood score of 33 and therapy tool B for users with a depression mood score around 71. This is a simple example for illustrative purposes, and the inner workings of the trained personalization model may be much more complex and take into account many more factors.

Process 300 is depicted with certain blocks in certain orders, although that need not always be the case. In some cases, process 300 can include fewer, additional, or different blocks, and in different orders.

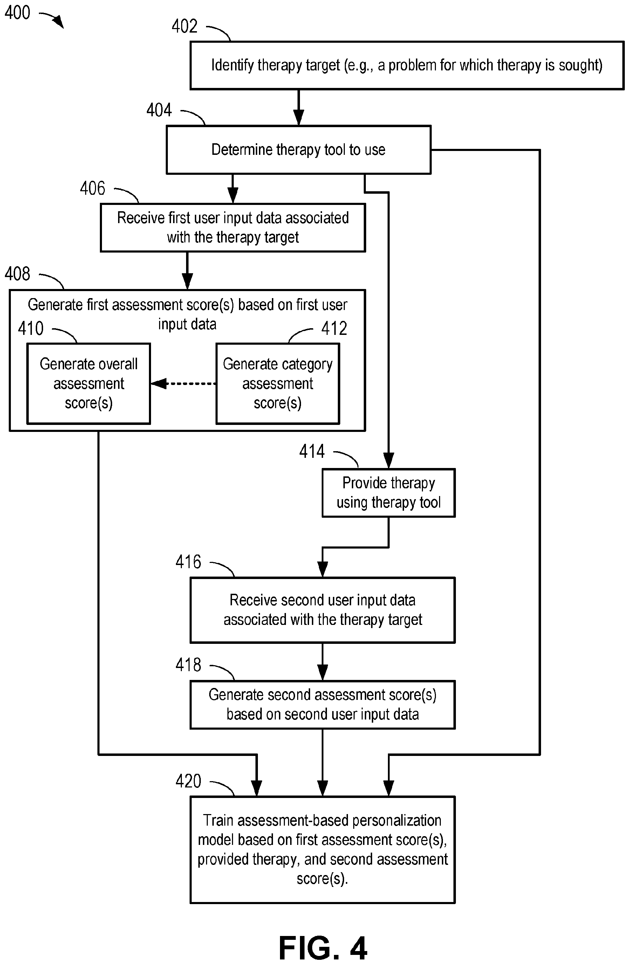

FIG. 4 is a flowchart depicting a process 400 for training an assessment-based personalization model, according to certain aspects of the present disclosure. Process 400 can be performed by any suitable computing device or computing devices, such as server(s) 106 of FIG. 1. Assessment-based personalization model can be any suitable model, such as the individual-trained personalization model from block 206 of FIG. 2.

At block 402, a therapy target (e.g., a problem for which therapy is sought). Identifying a therapy target can include receiving user input indicating a therapy target or suggesting a therapy target, or in some cases, can include accessing a list of one or more therapy targets already associated with the user.

At block 404, a therapy tool to be used can be determined. In some cases, determining the therapy tool to be used can include receiving user input selecting a particular therapy tool to be used. In some cases, determining a therapy tool to be used can include automatically selecting a therapy tool, such as described with reference to process 200 and block 220 of FIG. 2. Other techniques can be used.

At block 406, first user input data associated with the therapy target can be received. This first user input data can be used at block 408 to generate one or more first assessment score(s).

In some cases, generating the assessment score(s) at block 408 can include generating an overall assessment score at block 410 and/or generating one or more category assessment scores at block 412. An overall assessment score is indicative of the user's overall severity of therapy targets or conditions, such as an overall quality of mental health, and not the severity of any one therapy target. Each category assessment score, on the other hand, is indicative of the intensity or severity of a specific therapy target (e.g., depression, difficulty sleeping, anxiety, etc.).

An overall assessment score can be generated at block 410 i) based directly on the user input data from block 406; ii) based on one or more category assessment scores generated at block 412; or iii) based on a combination of i and ii. In an example of direct generation of the overall assessment score, in response to a question asking "how are you doing today, on a scale of 1 to 10?" the user may say "7," in which case the overall assessment score may be set to 7 out of 10, 70 out of 700, or some other comparable value. In an example of generating an overall assessment score based on one or more category assessment scores, the system may generate three category assessment scores, as described in further detail herein, with the values of 37, 42, and 89, all out of 100. In such an example, the overall assessment score may be an average of the values, or 56 out of 100.

At block 412, one or more category assessment scores can be generated. Each category assessment score is associated with a particular therapy target. In some cases, a single category assessment score can be further broken down into multiple additional category assessment scores. Generating a category assessment score at block 412 can include analyzing the first user input data from block 406 to calculate and/or update a category assessment score. For example, in response to a question asking "how often have you had little interest or pleasure in doing things in the past two weeks?" the user may select "Nearly every day (4)," in which case the category assessment score for depression (e.g., a depression assessment score or a depression score) can be set to a high level or otherwise increased. In some cases, a category assessment score can be based on direct input (e.g., a user ranking depression on a scale of 1 to 10) or can be interpreted from multiple questions and/or statements (e.g., a user filling out a Patient Health Questionnaire-9 survey, with the results being used to generate the category assessment score). In another example, the system may prompt the user to select one or more therapy targets that they are currently experiencing or currently wish to address. Based on the selection, the system may update the category assessment score(s) associated with the selected therapy target(s), as well as other category assessment score(s) that have a relationship to the selected therapy target(s). For example, if the user says they want to work on difficulty sleeping, the system may increment both a category assessment score for difficulty sleeping and a category assessment score for depression.

At block 414, therapy can be provided using the therapy tool determined at block 404. Providing therapy can include providing therapy as disclosed herein, such as via a chatbot, although that need not always be the case. The first assessment score(s) from block 408 are intended to capture the user's assessment(s) prior to the therapy provided at block 414. Thus, in some cases blocks 406, 408, 410, and 412 occur before block 414. In other cases, however, the user input data received at block 406 is provided prior to block 414, in which case the first assessment score(s) derived from that user input data can be generated at any time, including after block 414.

At block 416, second user input data is received. Receiving second user input data at block 416 can be similar to receiving first user input data at block 406. The second user input data is similar to first user input data, but associated with an assessment after therapy has been provided at block 414.

At block 418, second assessment score(s) can be generated at block 418. Generating second assessment score(s) at block 418 can be similar to generating first assessment score(s) at block 408. The second assessment score(s) are similar to the first assessment score(s), but associated with an assessment after therapy has been provided at block 414.

At block 420, an assessment-based personalization model can be trained. The assessment-based personalization model can be trained based at least in part on the first assessment score(s) from block 408, the second assessment score(s) from block 418, and the determined therapy tool from block 404, collectively known as training data. The training data can be collected from a single individual or from multiple individuals (e.g., a corpus of individuals). When collected from a single individual, training the assessment-based personalization model results in an individual-trained model. When collected from multiple individuals (e.g., from a cohort), the assessment-based personalization model results in a cohort-based personalization model. In such cases, optional cohort information can be provided to block 420, similar to as described with reference to block 318 of FIG. 3.

Training a model at block 420 can include using the training data (e.g., the first assessment score(s) from block 408, the second assessment score(s) from block 418, and the determined therapy tool from block 404) to generate desired outputs. Blocks 402, 404, 406, 408, 410, 412, 414, 416, 418 can be repeated multiple times (e.g., over the course of days, weeks, months, or years) to provide additional training data to train the assessment-based personalization model at block 420. The desired outputs can be set depending on the needs of the model. Desired outputs include those discussed with reference to block 216 of FIG. 2. For example, an assessment-based personalization model can be trained to identify the optimal therapy tool to use for a given set of assessment scores. In an example, training the personalization model can include setting a goal of achieving the largest improvement in an uncontrollable anxiety therapy target, which can be determined by comparing the first assessment score(s) with the second assessment score(s). As the training data is processed, the model can be adjusted until is able to output, for any given set of inputs (e.g., a set of current assessment score(s) for one or more therapy targets of the user), the proper therapy tool expected to achieve the largest improvement in the user's uncontrollable anxiety therapy target. For example, if the training data shows that when the user previously experienced category assessment scores for an uncontrollable anxiety therapy target around 80, therapy tool A ended up causing the largest improvement in that score, and when the score started around 20, therapy tool B ended up causing the largest improvement in that score, the output from the trained personalization model should indicate therapy tool A when the user's category assessment scores for an uncontrollable anxiety therapy target are 77, and therapy tool B when the user's score is 24. This is a simple example for illustrative purposes, and the inner workings of the trained personalization model may be much more complex and take into account many more factors.

Process 400 is depicted with certain blocks in certain orders, although that need not always be the case. In some cases, process 400 can include fewer, additional, or different blocks, and in different orders. For example, in some cases, blocks 406, 408, 410, and 412 may occur before block 404.

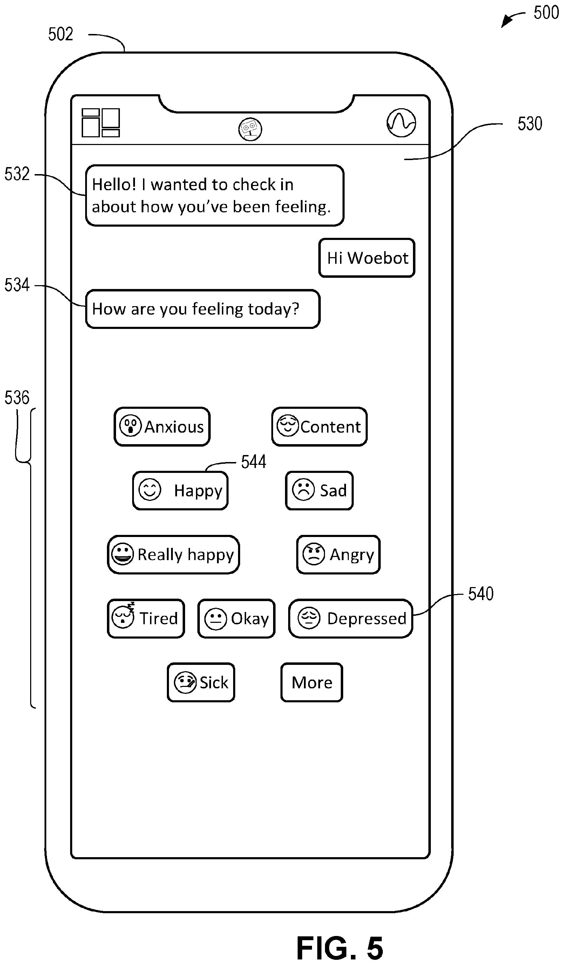

FIG. 5 is a schematic diagram depicting an example conversation path 500 for obtaining mood information on a graphical user interface (GUI) 530, according to certain aspects of the present disclosure. The GUI 530 can run on a user device 502, such as a smartphone. Any suitable user device 502 can be used, such as user device 102 of FIG. 1. The GUI 530 can present an interface for interacting with a chatbot. In some cases, processing for the chatbot occurs on one or more servers communicatively coupled to the user device 502, although that need not always be the case.

The conversation path 500 can include a first chat prompt 532 greeting the user. A second chat prompt 534 can request information from the user. Depending on the user's response to the second chat prompt 534, the chatbot may be able to generate (e.g., create or update) one or more mood score(s) and/or assessment score(s). As depicted in FIG. 5, the system provides the user with a set of possible responses 536 from which the user may pick a response. For example, if the user is happy, the user may select the happy response 544, but if the user is depressed, the user may select the depressed response 540.

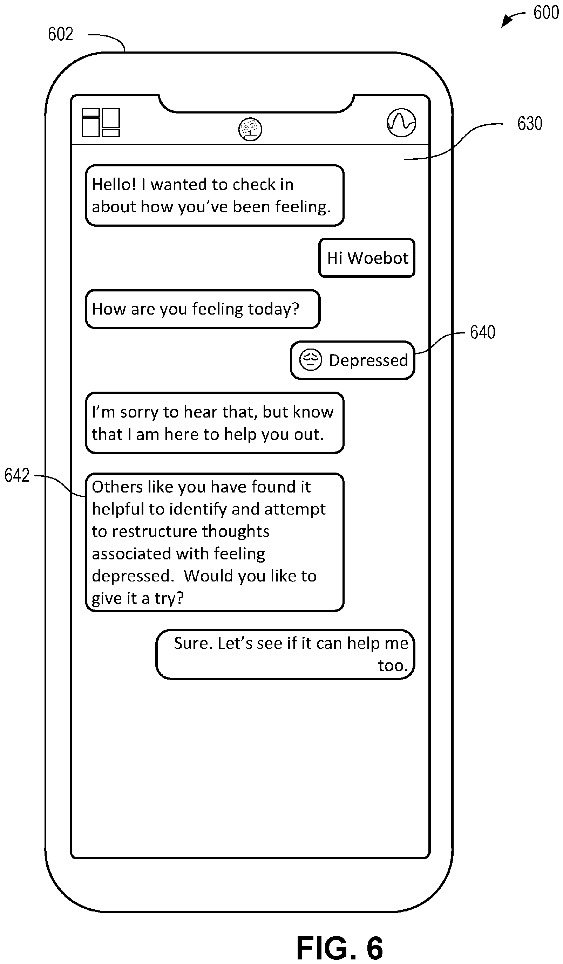

FIG. 6 is a schematic diagram depicting an example conversation path 600 on a graphical user interface 630 for a depressed response 640, according to certain aspects of the present disclosure. The GUI 630 can run on a user device 602, such as a smartphone. Any suitable user device 602 can be used, such as user device 102 of FIG. 1. The GUI 530 can present an interface for interacting with a chatbot. Conversation path 600 can be a continuation of conversation path 500 of FIG. 5.

In response to the depressed response 640, the system may generate one or more mood score(s) and/or assessment score(s) using the depressed response 640, then identify a therapy tool to use based on the generated score(s), such as via process 200 of FIG. 2. Once the appropriate therapy tool has been identified, the chatbot can present a third chat prompt acknowledging the user's response, then present a fourth chat prompt 642 asking the user if they would like to perform the therapy tool that was identified. While the therapy tool selected in conversation path 600 is restructuring thoughts, a user with one or more different mood score(s) and/or assessment score(s) (or in a different cohort) may be provided with a different therapy tool. Thus, the selection of therapy tool is personalized for each user based on that user's current needs.

While depicted with reference to selecting a therapy tool, the chatbot may be performing other tasks to personalize the user's therapy, such as establishing a therapy sequencing and/or therapy timing.

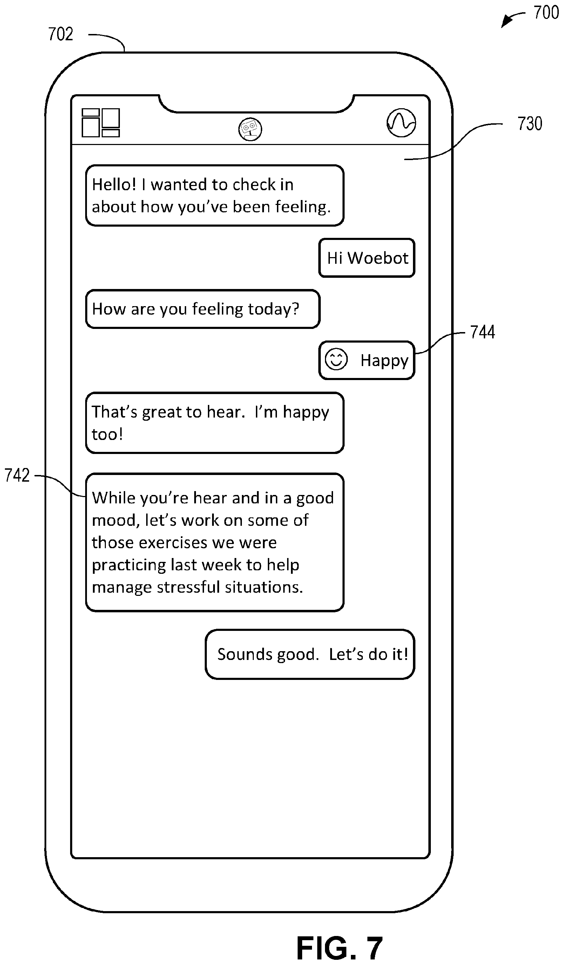

FIG. 7 is a schematic diagram depicting an example conversation path 700 on a graphical user interface 730 for a happy response 744, according to certain aspects of the present disclosure. The GUI 730 can run on a user device 702, such as a smartphone. Any suitable user device 702 can be used, such as user device 102 of FIG. 1. The GUI 730 can present an interface for interacting with a chatbot. Conversation path 700 can be a continuation of conversation path 500 of FIG. 5. Conversation path 700 can be an alternate version of conversation path 600 of FIG. 6 with selection of a different response.

In response to the happy response 744, the system may generate one or more mood score(s) and/or assessment score(s) using the happy response 744, then identify a therapy tool to use based on the generated score(s), such as via process 200 of FIG. 2. Once the appropriate therapy tool has been identified, the chatbot can present a third chat prompt acknowledging the user's response, then present a fourth chat prompt 742 asking the user if they would like to perform the therapy tool that was identified. While the therapy tool selected in conversation path 700 is practicing stress management exercises, a user with one or more different mood score(s) and/or assessment score(s) (or in a different cohort) may be provided with a different therapy tool. Thus, the selection of therapy tool is personalized for each user based on that user's current needs.

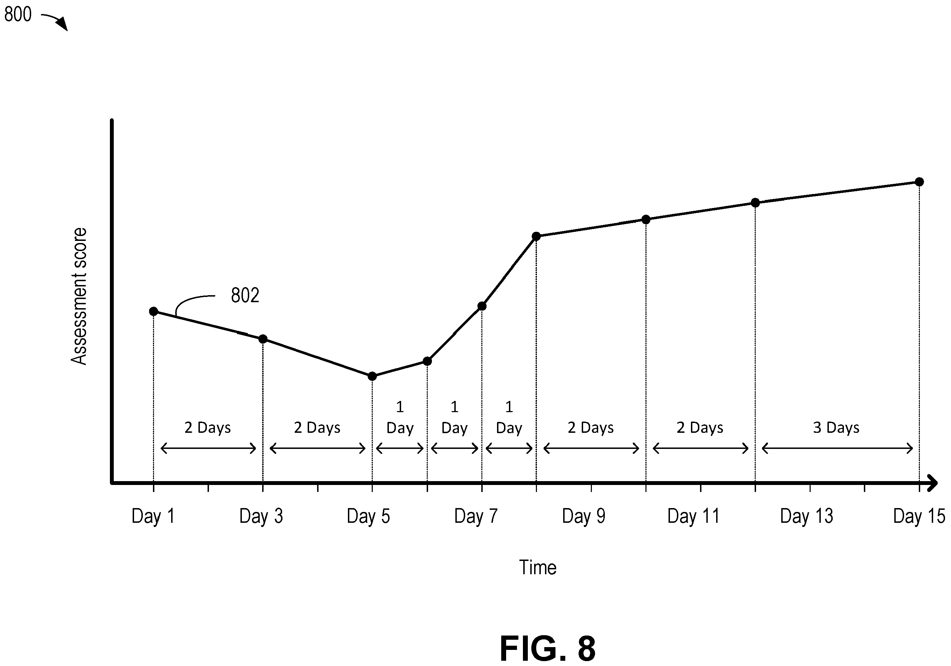

FIG. 8 is a chart 800 depicting how an assessment score affects therapy timing over time, according to certain aspects of the present disclosure. Chart 800 tracks an assessment score (e.g., an overall assessment score or a category assessment score) for an example user over a number of days. The user makes use of a chatbot-based automated therapy system, such as the therapy-providing system described with reference to FIG. 1.

The assessment score shown may be an assessment score acquire before use of a particular therapy tool. Each dot represents an instance where an assessment score was acquired and therapy was performed. Thus, for the therapy provided on Day 1, the assessment score on Day 1 acts as a pre-therapy assessment score and the assessment score on Day 3 acts as the post-therapy assessment score.

On Day 1, the user begins engaging with the therapy-providing system and receives therapy at a frequency of once every two days. On Day 3, the assessment score is shown to have dropped slightly. On Day 5, the assessment score is shown to have dropped further and by a larger amount. Because the user has shown a particular pattern of low and/or declining assessment scores (e.g., a particular assessment score trend), the personalization model can use the assessment score(s) to automatically determine a new therapy timing for the user, which is to receive therapy once per day.

On Day 6, after only a single day has elapsed, the assessment score is slightly higher. On Day 7, the score is significantly higher. On Day 8, the score is even higher. Because the user has shown a particular pattern of high and/or increasing assessment scores (e.g., a particular assessment score trend), the personalization model can use the assessment score(s) to automatically determine a new therapy timing for the user, which is to receive therapy once every two days (like the frequency used between Days 1 and 5).

On Day 10, the assessment score is shown to be slightly higher. On Day 12, the assessment score is shown to be slightly higher, still. At this point, because the user has shown a particular pattern of high and/or increasing assessment scores (e.g., a particular assessment score trend), the personalization model can use the assessment score(s) to automatically determine a new therapy timing for the user, which is to receive therapy once every three days. Thus, the next therapy is provided on Day 15 instead of Day 14.

The determination of when to change therapy timing and/or what the new therapy timing should be can be made by the personalization model. In some cases, threshold scores or threshold trends (e.g., a change of more than a threshold amount or threshold percentage) can be used to trigger when a new therapy timing should be obtained. In some cases, however, the therapy timing can be obtained each time (e.g., each Day), even if it is the same therapy timing as immediately previously used.

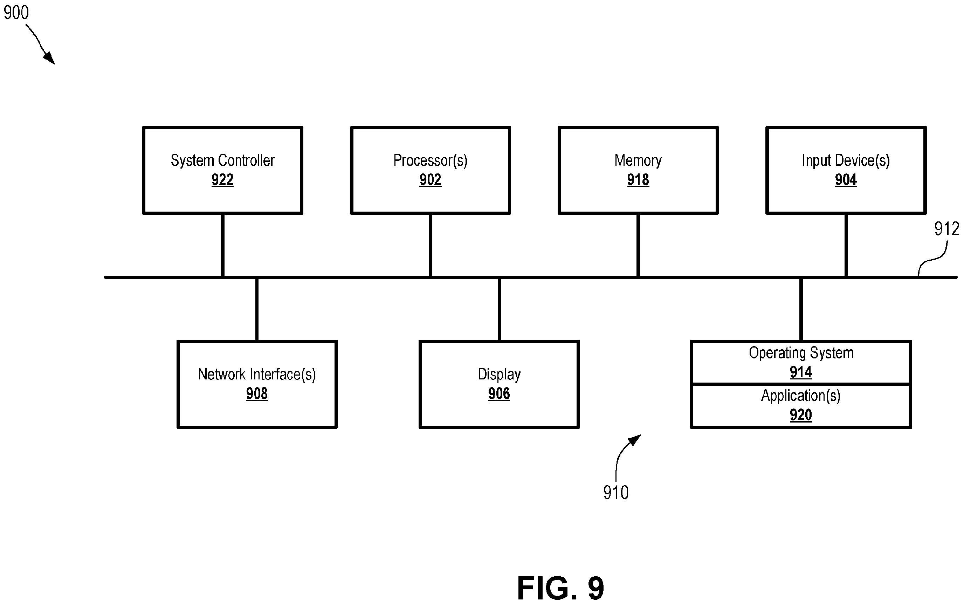

FIG. 9 is a block diagram of an example system architecture 900 for implementing features and processes of the present disclosure, such as those presented with reference to processes 200, 300, and 400 of FIGs. 2, 3, and 4, respectively. The architecture 900 can be used to implement a server (e.g., server 106 of FIG. 1), a user device (e.g., user device 102 of FIG. 1), or any other suitable device for performing some or all of the aspects of the present disclosure. The architecture 900 can be implemented on any electronic device that runs software applications derived from compiled instructions, including without limitation personal computers, servers, smart phones, electronic tablets, game consoles, email devices, and the like. In some implementations, the architecture 900 can include one or more processors 902, one or more input devices 904, one or more display devices 906, one or more network interfaces 908, and one or more computer-readable mediums 910. Each of these components can be coupled by bus 912.

Display device 906 can be any known display technology, including but not limited to display devices using Liquid Crystal Display (LCD) or Light Emitting Diode (LED) technology. Processor(s) 902 can use any known processor technology, including but not limited to graphics processors and multi-core processors. Input device 904 can be any known input device technology, including but not limited to a keyboard (including a virtual keyboard), mouse, track ball, and touch-sensitive pad or display. In some cases, audio inputs can be used to provide audio signals, such as audio signals of an individual speaking. Bus 912 can be any known internal or external bus technology, including but not limited to ISA, EISA, PCI, PCI Express, NuBus, USB, Serial ATA or FireWire.

Computer-readable medium 910 can be any medium that participates in providing instructions to processor(s) 902 for execution, including without limitation, non-volatile storage media (e.g., optical disks, magnetic disks, flash drives, etc.) or volatile media (e.g., SDRAM, ROM, etc.). The computer-readable medium (e.g., storage devices, mediums, and memories) can include, for example, a cable or wireless signal containing a bit stream and the like. However, when mentioned, non-transitory computer-readable storage media expressly exclude media such as energy, carrier signals, electromagnetic waves, and signals per se.

Computer-readable medium 910 can include various instructions for implementing operating system 914 and applications 920 such as computer programs. The operating system can be multi-user, multiprocessing, multitasking, multithreading, real-time and the like. The operating system 914 performs basic tasks, including but not limited to: recognizing input from input device 904; sending output to display device 906; keeping track of files and directories on computer-readable medium 910; controlling peripheral devices (e.g., storage drives, interface devices, etc.) which can be controlled directly or through an I/O controller; and managing traffic on bus 912. Computer-readable medium 910 can include various instructions for implementing firmware processes, such as a BIOS. Computer-readable medium 910 can include various instructions for implementing any of the processes described herein, including at least processes 200, 300, and 400 of FIGs. 2, 3, and 4, respectively.

Memory 918 can include high-speed random access memory and/or non-volatile memory, such as one or more magnetic disk storage devices, one or more optical storage devices, and/or flash memory (e.g., NAND, NOR). The memory 918 (e.g., computer-readable storage devices, mediums, and memories) can include a cable or wireless signal containing a bit stream and the like. However, when mentioned, non-transitory computer-readable storage media expressly exclude media such as energy, carrier signals, electromagnetic waves, and signals per se. The memory 918 can store an operating system, such as Darwin, RTXC, LINUX, UNIX, OS X, WINDOWS, or an embedded operating system such as VxWorks.

System controller 922 can be a service processor that operates independently of processor 902. In some implementations, system controller 922 can be a baseboard management controller (BMC). For example, a BMC is a specialized service processor that monitors the physical state of a computer, network server, or other hardware device using sensors and communicating with the system administrator through an independent connection. The BMC is configured on the motherboard or main circuit board of the device to be monitored. The sensors of a BMC can measure internal physical variables such as temperature, humidity, power-supply voltage, fan speeds, communications parameters and operating system (OS) functions.

The described features can be implemented advantageously in one or more computer programs that are executable on a programmable system including at least one programmable processor coupled to receive data and instructions from, and to transmit data and instructions to, a data storage system, at least one input device, and at least one output device. A computer program is a set of instructions that can be used, directly or indirectly, in a computer to perform a certain activity or bring about a certain result. A computer program can be written in any form of programming language (e.g., Objective-C, Java), including compiled or interpreted languages, and it can be deployed in any form, including as a stand-alone program or as a module, component, subroutine, or other unit suitable for use in a computing environment.

Suitable processors for the execution of a program of instructions include, by way of example, both general and special purpose microprocessors, and the sole processor or one of multiple processors or cores, of any kind of computer. Generally, a processor will receive instructions and data from a read-only memory or a random access memory or both. The essential elements of a computer are a processor for executing instructions and one or more memories for storing instructions and data. Generally, a computer will also include, or be operatively coupled to communicate with, one or more mass storage devices for storing data files; such devices include magnetic disks, such as internal hard disks and removable disks; magneto-optical disks; and optical disks. Storage devices suitable for tangibly embodying computer program instructions and data include all forms of non-volatile memory, including by way of example semiconductor memory devices, such as EPROM, EEPROM, and flash memory devices; magnetic disks such as internal hard disks and removable disks; magneto-optical disks; and CD-ROM and DVD-ROM disks. The processor and the memory can be supplemented by, or incorporated in, ASICs (application-specific integrated circuits).

To provide for interaction with a user, the features can be implemented on a computer having a display device such as a CRT (cathode ray tube) or LCD (liquid crystal display) monitor for displaying information to the user and a keyboard and a pointing device such as a mouse or a trackball by which the user can provide input to the computer.

The features can be implemented in a computing system that includes a back-end component, such as a data server, or that includes a middleware component, such as an application server or an Internet server, or that includes a front-end component, such as a client computer having a graphical user interface or an Internet browser, or any combination thereof. The components of the system can be connected by any form or medium of digital data communication such as a communication network. Examples of communication networks include, e.g., a LAN, a WAN, and the computers and networks forming the Internet.

The computing system can include clients and servers. A client and server are generally remote from each other and typically interact through a network. The relationship of client and server arises by virtue of computer programs running on the respective computers and having a client-server relationship to each other.

One or more features or steps of the disclosed embodiments can be implemented using an application programming interface (API). An API can define one or more parameters that are passed between a calling application and other software code (e.g., an operating system, library routine, function) that provides a service, that provides data, or that performs an operation or a computation.

The API can be implemented as one or more calls in program code that send or receive one or more parameters through a parameter list or other structure based on a call convention defined in an API specification document. A parameter can be a constant, a key, a data structure, an object, an object class, a variable, a data type, a pointer, an array, a list, or another call. API calls and parameters can be implemented in any programming language. The programming language can define the vocabulary and calling convention that a programmer will employ to access functions supporting the API.

In some implementations, an API call can report to an application the capabilities of a device running the application, such as input capability, output capability, processing capability, power capability, communications capability, and the like.

The foregoing description of the embodiments, including illustrated embodiments, has been presented only for the purpose of illustration and description and is not intended to be exhaustive or limiting to the precise forms disclosed. Numerous modifications, adaptations, and uses thereof will be apparent to those skilled in the art. Numerous changes to the disclosed embodiments can be made in accordance with the disclosure herein, without departing from the spirit or scope of the disclosure. Thus, the breadth and scope of the present disclosure should not be limited by any of the above described embodiments.

Although certain aspects and features of the present disclosure have been illustrated and described with respect to one or more implementations, equivalent alterations and modifications will occur or be known to others skilled in the art upon the reading and understanding of this specification and the annexed drawings. In addition, while a particular feature may have been disclosed with respect to only one of several implementations, such feature may be combined with one or more other features of the other implementations as may be desired and advantageous for any given or particular application.

The terminology used herein is for the purpose of describing particular embodiments only, and is not intended to be limiting of the disclosure. As used herein, the singular forms "a," "an," and "the" are intended to include the plural forms as well, unless the context clearly indicates otherwise. Furthermore, to the extent that the terms "including," "includes," "having," "has," "with," or variants thereof, are used in either the detailed description and/or the claims, such terms are intended to be inclusive in a manner similar to the term "comprising."

As used below, any reference to a series of examples is to be understood as a reference to each of those examples disjunctively (e.g., "Examples 1-4" is to be understood as "Examples 1, 2, 3, or 4").

Example 1 is a system, comprising: one or more data processors; and a non-transitory computer-readable storage medium containing instructions which, when executed on the one or more data processors, cause the one or more data processors to perform operations including: receiving cohort information associated with a user; receiving mood information associated with the user, wherein receiving the mood information includes receiving user input data and generating one or more mood scores based at least in part on the input data; accessing a mood-based personalization model based on the cohort information, wherein the mood-based personalization model is trained, at least in part, using training data for a plurality individuals associated with the cohort, wherein the training data includes a plurality of mood scores associated with the plurality of individuals associated with the cohort; determining a therapy tool to be used by applying the one or more mood scores to the mood-based personalization model; and facilitating providing personalized therapy to the user using the determined therapy tool.

Example 2 is the system of example(s) 1, wherein determining the therapy tool includes selecting a therapy tool out of a plurality of possible therapy tools.

Example 3 is the system of example(s) 1 or 2, wherein determining the therapy tool includes: generating a therapy tool effectiveness score for each of the plurality of possible therapy tools based at least in part on applying the one or more mood scores to the mood-based personalization model; ranking each of the plurality of possible therapy tools based on the respective therapy tool effectiveness score; and selecting one of the plurality of possible therapy tools based on the ranking.

Example 4 is the system of example(s) 1-3, wherein receiving cohort information associated with the user includes: receiving additional user input data; and assigning the user to the cohort based at least in part on the additional user input data.

Example 5 is the system of example(s) 1-4, wherein generating one or more mood scores includes generating an overall mood score, wherein the overall mood score is indicative of a positive mood or a negative mood, and wherein the overall mood score is further indicative of a strength of the positive mood or the negative mood.

Example 6 is the system of example(s) 1-5, wherein generating one or more mood scores includes generating one or more enumerated mood scores, wherein each of the one or more enumerated mood scores is associated with a unique mood, and wherein each of the one or more enumerated mood scores is indicative of a strength of its respective unique mood; wherein the plurality of mood scores associated with the plurality of individuals associated with the cohort includes a plurality of enumerated mood scores associated with the plurality of individuals associated with the cohort; and wherein determining the therapy tool to be used by applying the one or more mood scores to the mood-based personalization model includes applying each of the one or more enumerated mood scores to the mood-based personalization model.

Example 7 is the system of example(s) 1-6, wherein generating one or more mood scores includes: generating one or more enumerated mood scores, wherein each of the one or more enumerated mood scores is associated with a unique mood, and wherein each of the one or more enumerated mood scores is indicative of a strength of its respective unique mood; and generating an overall mood score based at least in part on the one or more enumerated mood scores.

Example 8 is the system of example(s) 1-7, wherein facilitating providing personalized therapy includes presenting a prompt to begin therapy using the determined therapy tool.

Example 9 is the system of example(s) 1-8, wherein receiving the user input data includes: establishing, via a network interface, a chat interface with a user device associated with the user; and receiving, via the chat interface, the user input data, wherein facilitating providing the personalized therapy includes initiating the therapy tool via the chat interface.

Example 10 is the system of example(s) 1-9, wherein the mood-based personalization model is further trained using additional training data associated with the user, wherein the additional training data includes historical mood information associated with historical therapy tool information, wherein the historical therapy tool information is indicative of at least one historical use of one or more therapy tools, and wherein the historical mood information is indicative of i) at least one historical mood score that occurred prior to one of the at least one historical use of the one or more therapy tools; ii) at least one historical mood score that occurred after the one of the at least one historical use of the one or more therapy tools; or iii) a combination of i and ii.

Example 11 is a computer-implemented method, comprising: receiving cohort information associated with a user; receiving mood information associated with the user, wherein receiving the mood information includes receiving user input data and generating one or more mood scores based at least in part on the input data; accessing a mood-based personalization model based on the cohort information, wherein the mood-based personalization model is trained, at least in part, using training data for a plurality individuals associated with the cohort, wherein the training data includes a plurality of mood scores associated with the plurality of individuals associated with the cohort; determining a therapy tool to be used by applying the one or more mood scores to the mood-based personalization model; and facilitating providing personalized therapy to the user using the determined therapy tool.

Example 12 is the computer-implemented method of example(s) 11, wherein determining the therapy tool includes selecting a therapy tool out of a plurality of possible therapy tools.

Example 13 is the computer-implemented method of example(s) 11 or 12, wherein determining the therapy tool includes: generating a therapy tool effectiveness score for each of the plurality of possible therapy tools based at least in part on applying the one or more mood scores to the mood-based personalization model; ranking each of the plurality of possible therapy tools based on the respective therapy tool effectiveness score; and selecting one of the plurality of possible therapy tools based on the ranking.

Example 14 is the computer-implemented method of example(s) 11-13, wherein receiving cohort information associated with the user includes: receiving additional user input data; and assigning the user to the cohort based at least in part on the additional user input data.

Example 15 is the computer-implemented method of example(s) 11-14, wherein generating one or more mood scores includes generating an overall mood score, wherein the overall mood score is indicative of a positive mood or a negative mood, and wherein the overall mood score is further indicative of a strength of the positive mood or the negative mood.

Example 16 is the computer-implemented method of example(s) 11-15, wherein generating one or more mood scores includes generating one or more enumerated mood scores, wherein each of the one or more enumerated mood scores is associated with a unique mood, and wherein each of the one or more enumerated mood scores is indicative of a strength of its respective unique mood; wherein the plurality of mood scores associated with the plurality of individuals associated with the cohort includes a plurality of enumerated mood scores associated with the plurality of individuals associated with the cohort; and wherein determining the therapy tool to be used by applying the one or more mood scores to the mood-based personalization model includes applying each of the one or more enumerated mood scores to the mood-based personalization model.

Example 17 is the computer-implemented method of example(s) 11-16, wherein generating one or more mood scores includes: generating one or more enumerated mood scores, wherein each of the one or more enumerated mood scores is associated with a unique mood, and wherein each of the one or more enumerated mood scores is indicative of a strength of its respective unique mood; and generating an overall mood score based at least in part on the one or more enumerated mood scores.

Example 18 is the computer-implemented method of example(s) 11-17, wherein facilitating providing personalized therapy includes presenting a prompt to begin therapy using the determined therapy tool.

Example 19 is the computer-implemented method of example(s) 11-18, wherein receiving the user input data includes: establishing, via a network interface, a chat interface with a user device associated with the user; and receiving, via the chat interface, the user input data, wherein facilitating providing the personalized therapy includes initiating the therapy tool via the chat interface.

Example 20 is the computer-implemented method of example(s) 11-19, wherein the mood-based personalization model is further trained using additional training data associated with the user, wherein the additional training data includes historical mood information associated with historical therapy tool information, wherein the historical therapy tool information is indicative of at least one historical use of one or more therapy tools, and wherein the historical mood information is indicative of i) at least one historical mood score that occurred prior to one of the at least one historical use of the one or more therapy tools; ii) at least one historical mood score that occurred after the one of the at least one historical use of the one or more therapy tools; or iii) a combination of i and ii.

Example 21 is a computer-program product tangibly embodied in a non-transitory machine-readable storage medium, including instructions configured to cause a data processing apparatus to perform operations including: receiving cohort information associated with a user; receiving mood information associated with the user, wherein receiving the mood information includes receiving user input data and generating one or more mood scores based at least in part on the input data; accessing a mood-based personalization model based on the cohort information, wherein the mood-based personalization model is trained, at least in part, using training data for a plurality individuals associated with the cohort, wherein the training data includes a plurality of mood scores associated with the plurality of individuals associated with the cohort; determining a therapy tool to be used by applying the one or more mood scores to the mood-based personalization model; and facilitating providing personalized therapy to the user using the determined therapy tool.

Example 22 is the computer-program product of example(s) 21, wherein determining the therapy tool includes selecting a therapy tool out of a plurality of possible therapy tools.

Example 23 is the computer-program product of example(s) 21 or 22, wherein determining the therapy tool includes: generating a therapy tool effectiveness score for each of the plurality of possible therapy tools based at least in part on applying the one or more mood scores to the mood-based personalization model; ranking each of the plurality of possible therapy tools based on the respective therapy tool effectiveness score; and selecting one of the plurality of possible therapy tools based on the ranking.

Example 24 is the computer-program product of example(s) 21-23, wherein receiving cohort information associated with the user includes: receiving additional user input data; and assigning the user to the cohort based at least in part on the additional user input data.

Example 25 is the computer-program product of example(s) 21-24, wherein generating one or more mood scores includes generating an overall mood score, wherein the overall mood score is indicative of a positive mood or a negative mood, and wherein the overall mood score is further indicative of a strength of the positive mood or the negative mood.

Example 26 is the computer-program product of example(s) 21-25, wherein generating one or more mood scores includes generating one or more enumerated mood scores, wherein each of the one or more enumerated mood scores is associated with a unique mood, and wherein each of the one or more enumerated mood scores is indicative of a strength of its respective unique mood; wherein the plurality of mood scores associated with the plurality of individuals associated with the cohort includes a plurality of enumerated mood scores associated with the plurality of individuals associated with the cohort; and wherein determining the therapy tool to be used by applying the one or more mood scores to the mood-based personalization model includes applying each of the one or more enumerated mood scores to the mood-based personalization model.

Example 27 is the computer-program product of example(s) 21-26, wherein generating one or more mood scores includes: generating one or more enumerated mood scores, wherein each of the one or more enumerated mood scores is associated with a unique mood, and wherein each of the one or more enumerated mood scores is indicative of a strength of its respective unique mood; and generating an overall mood score based at least in part on the one or more enumerated mood scores.

Example 28 is the computer-program product of example(s) 21-27, wherein facilitating providing personalized therapy includes presenting a prompt to begin therapy using the determined therapy tool.

Example 29 is the computer-program product of example(s) 21-28, wherein receiving the user input data includes: establishing, via a network interface, a chat interface with a user device associated with the user; and receiving, via the chat interface, the user input data, wherein facilitating providing the personalized therapy includes initiating the therapy tool via the chat interface.

Example 30 is the computer-program product of example(s) 21-29, wherein the mood-based personalization model is further trained using additional training data associated with the user, wherein the additional training data includes historical mood information associated with historical therapy tool information, wherein the historical therapy tool information is indicative of at least one historical use of one or more therapy tools, and wherein the historical mood information is indicative of i) at least one historical mood score that occurred prior to one of the at least one historical use of the one or more therapy tools; ii) at least one historical mood score that occurred after the one of the at least one historical use of the one or more therapy tools; or iii) a combination of i and ii.

Example 31 is a system, comprising: one or more data processors; and a non-transitory computer-readable storage medium containing instructions which, when executed on the one or more data processors, cause the one or more data processors to perform operations including: receiving cohort information associated with a user; receiving mood information associated with the user, wherein receiving the mood information includes receiving user input data and generating one or more mood scores based at least in part on the input data; accessing a mood-based personalization model based on the cohort information, wherein the mood-based personalization model is trained, at least in part, using training data for a plurality individuals associated with the cohort, wherein the training data includes a plurality of mood scores associated with the plurality of individuals associated with the cohort; determining therapy sequencing to be used by applying the one or more mood scores to the mood-based personalization model, wherein the determined therapy sequencing is indicative of i) an order for applying a plurality of therapy tools; ii) an order for addressing therapy targets; or iii) a combination of i and ii; and facilitating providing personalized therapy to the user using the determined therapy sequencing.

Example 32 is the system of example(s) 31, wherein determining the therapy sequencing includes: identifying a priority therapy target out of a plurality of potential therapy targets based at least in part on the one or more mood scores; and selecting a therapy sequencing that addresses the priority therapy target before others of the plurality of potential therapy targets.

Example 33 is the system of example(s) 31 or 32, wherein determining the therapy sequencing includes: identifying a low-priority therapy target out of a plurality of potential therapy targets based at least in part on the application of the one or more mood scores to the mood-based personalization model; and selecting a therapy sequencing that addresses others of the plurality of potential therapy targets before the low-priority therapy target.

Example 34 is the system of example(s) 31-33, wherein receiving cohort information associated with the user includes: receiving additional user input data; and assigning the user to the cohort based at least in part on the additional user input data.

Example 35 is the system of example(s) 31-34, wherein generating one or more mood scores includes generating an overall mood score, wherein the overall mood score is indicative of a positive mood or a negative mood, and wherein the overall mood score is further indicative of a strength of the positive mood or the negative mood.

Example 36 is the system of example(s) 31-35, wherein generating one or more mood scores includes generating one or more enumerated mood scores, wherein each of the one or more enumerated mood scores is associated with a unique mood, and wherein each of the one or more enumerated mood scores is indicative of a strength of its respective unique mood; wherein the plurality of mood scores associated with the plurality of individuals associated with the cohort includes a plurality of enumerated mood scores associated with the plurality of individuals associated with the cohort; and wherein determining the therapy sequencing to be used by applying the one or more mood scores to the mood-based personalization model includes applying each of the one or more enumerated mood scores to the mood-based personalization model.

Example 37 is the system of example(s) 36, wherein determining the therapy sequencing to be used by applying each of the one or more enumerated mood scores to the mood-based personalization model results in the determined therapy sequencing that is based at least in part on the intensity of each of the one or more unique moods.

Example 38 is the system of example(s) 31-37, wherein generating one or more mood scores includes: generating one or more enumerated mood scores, wherein each of the one or more enumerated mood scores is associated with a unique mood, and wherein each of the one or more enumerated mood scores is indicative of a strength of its respective unique mood; and generating an overall mood score based at least in part on the one or more enumerated mood scores.

Example 39 is the system of example(s) 31-38, wherein facilitating providing personalized therapy includes: presenting a first prompt to begin therapy using a first therapy tool based at least in part on the determined therapy sequencing; and presenting a second prompt, after completion of the first therapy tool; to begin therapy using a second therapy tool based at least in part on the determined therapy sequencing.

Example 40 is the system of example(s) 31-39, wherein receiving the user input data includes: establishing, via a network interface, a chat interface with a user device associated with the user; and receiving, via the chat interface, the user input data, wherein facilitating providing the personalized therapy includes initiating the therapy tool via the chat interface.

Example 41 is the system of example(s) 31-40, wherein the mood-based personalization model is further trained using additional training data associated with the user, wherein the additional training data includes historical mood information associated with historical therapy tool information, wherein the historical therapy tool information is indicative of a historical therapy sequencing of applying one or more therapy tools, and wherein the historical mood information is indicative of i) at least one historical mood score that occurred prior to the historical therapy sequencing; ii) at least one historical mood score that occurred during the historical therapy sequencing; iii) at least one historical mood score that occurred after the historical therapy sequencing; or iv) any combination of i to iii.

Example 42 is a computer-implemented method, comprising: receiving cohort information associated with a user; receiving mood information associated with the user, wherein receiving the mood information includes receiving user input data and generating one or more mood scores based at least in part on the input data; accessing a mood-based personalization model based on the cohort information, wherein the mood-based personalization model is trained, at least in part, using training data for a plurality individuals associated with the cohort, wherein the training data includes a plurality of mood scores associated with the plurality of individuals associated with the cohort; determining therapy sequencing to be used by applying the one or more mood scores to the mood-based personalization model, wherein the determined therapy sequencing is indicative of i) an order for applying a plurality of therapy tools; ii) an order for addressing therapy targets; or iii) a combination of i and ii; and facilitating providing personalized therapy to the user using the determined therapy sequencing.

Example 43 is the computer-implemented method of example(s) 42, wherein determining the therapy sequencing includes: identifying a priority therapy target out of a plurality of potential therapy targets based at least in part on the one or more mood scores; and selecting a therapy sequencing that addresses the priority therapy target before others of the plurality of potential therapy targets.

Example 44 is the computer-implemented method of example(s) 42 or 43, wherein determining the therapy sequencing includes: identifying a low-priority therapy target out of a plurality of potential therapy targets based at least in part on the application of the one or more mood scores to the mood-based personalization model; and selecting a therapy sequencing that addresses others of the plurality of potential therapy targets before the low-priority therapy target.

Example 45 is the computer-implemented method of example(s) 42-44, wherein receiving cohort information associated with the user includes: receiving additional user input data; and assigning the user to the cohort based at least in part on the additional user input data.

Example 46 is the computer-implemented method of example(s) 42-45, wherein generating one or more mood scores includes generating an overall mood score, wherein the overall mood score is indicative of a positive mood or a negative mood, and wherein the overall mood score is further indicative of a strength of the positive mood or the negative mood.

Example 47 is the computer-implemented method of example(s) 42-46, wherein generating one or more mood scores includes generating one or more enumerated mood scores, wherein each of the one or more enumerated mood scores is associated with a unique mood, and wherein each of the one or more enumerated mood scores is indicative of a strength of its respective unique mood; wherein the plurality of mood scores associated with the plurality of individuals associated with the cohort includes a plurality of enumerated mood scores associated with the plurality of individuals associated with the cohort; and wherein determining the therapy sequencing to be used by applying the one or more mood scores to the mood-based personalization model includes applying each of the one or more enumerated mood scores to the mood-based personalization model.

Example 48 is the computer-implemented method of example(s) 47, wherein determining the therapy sequencing to be used by applying each of the one or more enumerated mood scores to the mood-based personalization model results in the determined therapy sequencing that is based at least in part on the intensity of each of the one or more unique moods.

Example 49 is the computer-implemented method of example(s) 42-48, wherein generating one or more mood scores includes: generating one or more enumerated mood scores, wherein each of the one or more enumerated mood scores is associated with a unique mood, and wherein each of the one or more enumerated mood scores is indicative of a strength of its respective unique mood; and generating an overall mood score based at least in part on the one or more enumerated mood scores.

Example 50 is the computer-implemented method of example(s) 42-49, wherein facilitating providing personalized therapy includes: presenting a first prompt to begin therapy using a first therapy tool based at least in part on the determined therapy sequencing; and presenting a second prompt, after completion of the first therapy tool; to begin therapy using a second therapy tool based at least in part on the determined therapy sequencing.

Example 51 is the computer-implemented method of example(s) 42-50, wherein receiving the user input data includes: establishing, via a network interface, a chat interface with a user device associated with the user; and receiving, via the chat interface, the user input data, wherein facilitating providing the personalized therapy includes initiating the therapy tool via the chat interface.

Example 52 is the computer-implemented method of example(s) 42-51, wherein the mood-based personalization model is further trained using additional training data associated with the user, wherein the additional training data includes historical mood information associated with historical therapy tool information, wherein the historical therapy tool information is indicative of a historical therapy sequencing of applying one or more therapy tools, and wherein the historical mood information is indicative of i) at least one historical mood score that occurred prior to the historical therapy sequencing; ii) at least one historical mood score that occurred during the historical therapy sequencing; iii) at least one historical mood score that occurred after the historical therapy sequencing; or iv) any combination of i to iii.

Example 53 is a computer-program product tangibly embodied in a non-transitory machine-readable storage medium, including instructions configured to cause a data processing apparatus to perform operations including: receiving cohort information associated with a user; receiving mood information associated with the user, wherein receiving the mood information includes receiving user input data and generating one or more mood scores based at least in part on the input data; accessing a mood-based personalization model based on the cohort information, wherein the mood-based personalization model is trained, at least in part, using training data for a plurality individuals associated with the cohort, wherein the training data includes a plurality of mood scores associated with the plurality of individuals associated with the cohort; determining therapy sequencing to be used by applying the one or more mood scores to the mood-based personalization model, wherein the determined therapy sequencing is indicative of i) an order for applying a plurality of therapy tools; ii) an order for addressing therapy targets; or iii) a combination of i and ii; and facilitating providing personalized therapy to the user using the determined therapy sequencing.

Example 54 is the computer-program product of example(s) 53, wherein determining the therapy sequencing includes: identifying a priority therapy target out of a plurality of potential therapy targets based at least in part on the one or more mood scores; and selecting a therapy sequencing that addresses the priority therapy target before others of the plurality of potential therapy targets.

Example 55 is the computer-program product of example(s) 53 or 54, wherein determining the therapy sequencing includes: identifying a low-priority therapy target out of a plurality of potential therapy targets based at least in part on the application of the one or more mood scores to the mood-based personalization model; and selecting a therapy sequencing that addresses others of the plurality of potential therapy targets before the low-priority therapy target.

Example 56 is the computer-program product of example(s) 53-55, wherein receiving cohort information associated with the user includes: receiving additional user input data; and assigning the user to the cohort based at least in part on the additional user input data.

Example 57 is the computer-program product of example(s) 53-56, wherein generating one or more mood scores includes generating an overall mood score, wherein the overall mood score is indicative of a positive mood or a negative mood, and wherein the overall mood score is further indicative of a strength of the positive mood or the negative mood.

Example 58 is the computer-program product of example(s) 53-57, wherein generating one or more mood scores includes generating one or more enumerated mood scores, wherein each of the one or more enumerated mood scores is associated with a unique mood, and wherein each of the one or more enumerated mood scores is indicative of a strength of its respective unique mood; wherein the plurality of mood scores associated with the plurality of individuals associated with the cohort includes a plurality of enumerated mood scores associated with the plurality of individuals associated with the cohort; and wherein determining the therapy sequencing to be used by applying the one or more mood scores to the mood-based personalization model includes applying each of the one or more enumerated mood scores to the mood-based personalization model.

Example 59 is the computer-program product of example(s) 58, wherein determining the therapy sequencing to be used by applying each of the one or more enumerated mood scores to the mood-based personalization model results in the determined therapy sequencing that is based at least in part on the intensity of each of the one or more unique moods.

Example 60 is the computer-program product of example(s) 53-59, wherein generating one or more mood scores includes: generating one or more enumerated mood scores, wherein each of the one or more enumerated mood scores is associated with a unique mood, and wherein each of the one or more enumerated mood scores is indicative of a strength of its respective unique mood; and generating an overall mood score based at least in part on the one or more enumerated mood scores.

Example 61 is the computer-program product of example(s) 53-60, wherein facilitating providing personalized therapy includes: presenting a first prompt to begin therapy using a first therapy tool based at least in part on the determined therapy sequencing; and presenting a second prompt, after completion of the first therapy tool; to begin therapy using a second therapy tool based at least in part on the determined therapy sequencing.

Example 62 is the computer-program product of example(s) 53-61, wherein receiving the user input data includes: establishing, via a network interface, a chat interface with a user device associated with the user; and receiving, via the chat interface, the user input data, wherein facilitating providing the personalized therapy includes initiating the therapy tool via the chat interface.

Example 63 is the computer-program product of example(s) 53-62, wherein the mood-based personalization model is further trained using additional training data associated with the user, wherein the additional training data includes historical mood information associated with historical therapy tool information, wherein the historical therapy tool information is indicative of a historical therapy sequencing of applying one or more therapy tools, and wherein the historical mood information is indicative of i) at least one historical mood score that occurred prior to the historical therapy sequencing; ii) at least one historical mood score that occurred during the historical therapy sequencing; iii) at least one historical mood score that occurred after the historical therapy sequencing; or iv) any combination of i to iii.

Example 64 is a system, comprising: one or more data processors; and a non-transitory computer-readable storage medium containing instructions which, when executed on the one or more data processors, cause the one or more data processors to perform operations including: receiving cohort information associated with a user; receiving mood information associated with the user, wherein receiving the mood information includes receiving user input data and generating one or more mood scores based at least in part on the input data; accessing a mood-based personalization model based on the cohort information, wherein the mood-based personalization model is trained, at least in part, using training data for a plurality individuals associated with the cohort, wherein the training data includes a plurality of mood scores associated with the plurality of individuals associated with the cohort; determining therapy timing to be used by applying the one or more mood scores to the mood-based personalization model, wherein the determined therapy timing is indicative of i) a frequency for applying one or more therapy tools; ii) a future time to apply the one or more therapy tools; or iii) a combination of i and ii; and facilitating providing personalized therapy to the user using the determined therapy timing.

Example 65 is the system of example(s) 64, wherein determining the therapy timing includes: selecting a therapy tool to use out of the one or more therapy tools; and determining the frequency for applying the therapy tool by applying the one or more mood scores and the selected therapy tool to the mood-based personalization model, wherein the plurality of mood scores of the training data includes a set of mood scores that are associated with the therapy tool.

Example 66 is the system of example(s) 64 or 65, wherein receiving cohort information associated with the user includes: receiving additional user input data; and assigning the user to the cohort based at least in part on the additional user input data.

Example 67 is the system of example(s) 64-66, wherein generating one or more mood scores includes generating an overall mood score, wherein the overall mood score is indicative of a positive mood or a negative mood, and wherein the overall mood score is further indicative of a strength of the positive mood or the negative mood.

Example 68 is the system of example(s) 64-67, wherein generating one or more mood scores includes generating one or more enumerated mood scores, wherein each of the one or more enumerated mood scores is associated with a unique mood, and wherein each of the one or more enumerated mood scores is indicative of a strength of its respective unique mood; wherein the plurality of mood scores associated with the plurality of individuals associated with the cohort includes a plurality of enumerated mood scores associated with the plurality of individuals associated with the cohort; and wherein determining the therapy timing to be used by applying the one or more mood scores to the mood-based personalization model includes applying each of the one or more enumerated mood scores to the mood-based personalization model.

Example 69 is the system of example(s) 68, wherein determining the therapy timing to be used by applying each of the one or more enumerated mood scores to the mood-based personalization model results in the determined therapy timing that is based at least in part on the intensity of each of the one or more unique moods.

Example 70 is the system of example(s) 64-69, wherein generating one or more mood scores includes: generating one or more enumerated mood scores, wherein each of the one or more enumerated mood scores is associated with a unique mood, and wherein each of the one or more enumerated mood scores is indicative of a strength of its respective unique mood; and generating an overall mood score based at least in part on the one or more enumerated mood scores.

Example 71 is the system of example(s) 64-70, wherein facilitating providing personalized therapy includes: determining a first time to initiate therapy based at least in part on the determined therapy timing; presenting a first prompt to begin therapy at the first time; determining a second time to initiate therapy based at least in part on the determined therapy timing; and presenting a second prompt to begin therapy at the second time.

Example 72 is the system of example(s) 64-71, wherein receiving the user input data includes: establishing, via a network interface, a chat interface with a user device associated with the user; and receiving, via the chat interface, the user input data, wherein facilitating providing the personalized therapy includes initiating the therapy tool via the chat interface.

Example 73 is the system of example(s) 64-72, wherein the mood-based personalization model is further trained using additional training data associated with the user, wherein the additional training data includes historical mood information associated with historical therapy tool information, wherein the historical therapy tool information is indicative of a historical therapy timing of applying one or more therapy tools, and wherein the historical mood information is indicative of i) at least one historical mood score that occurred prior to the historical therapy timing; ii) at least one historical mood score that occurred during the historical therapy timing; iii) at least one historical mood score that occurred after the historical therapy timing; or iv) any combination of i to iii.

Example 74 is a computer-implemented method, comprising: receiving cohort information associated with a user; receiving mood information associated with the user, wherein receiving the mood information includes receiving user input data and generating one or more mood scores based at least in part on the input data; accessing a mood-based personalization model based on the cohort information, wherein the mood-based personalization model is trained, at least in part, using training data for a plurality individuals associated with the cohort, wherein the training data includes a plurality of mood scores associated with the plurality of individuals associated with the cohort; determining therapy timing to be used by applying the one or more mood scores to the mood-based personalization model, wherein the determined therapy timing is indicative of i) a frequency for applying one or more therapy tools; ii) a future time to apply the one or more therapy tools; or iii) a combination of i and ii; and facilitating providing personalized therapy to the user using the determined therapy timing.

Example 75 is the computer-implemented method of example(s) 74, wherein determining the therapy timing includes: selecting a therapy tool to use out of the one or more therapy tools; and determining the frequency for applying the therapy tool by applying the one or more mood scores and the selected therapy tool to the mood-based personalization model, wherein the plurality of mood scores of the training data includes a set of mood scores that are associated with the therapy tool.

Example 76 is the computer-implemented method of example(s) 74 or 75, wherein receiving cohort information associated with the user includes: receiving additional user input data; and assigning the user to the cohort based at least in part on the additional user input data.

Example 77 is the computer-implemented method of example(s) 74-76, wherein generating one or more mood scores includes generating an overall mood score, wherein the overall mood score is indicative of a positive mood or a negative mood, and wherein the overall mood score is further indicative of a strength of the positive mood or the negative mood.

Example 78 is the computer-implemented method of example(s) 74-77, wherein generating one or more mood scores includes generating one or more enumerated mood scores, wherein each of the one or more enumerated mood scores is associated with a unique mood, and wherein each of the one or more enumerated mood scores is indicative of a strength of its respective unique mood; wherein the plurality of mood scores associated with the plurality of individuals associated with the cohort includes a plurality of enumerated mood scores associated with the plurality of individuals associated with the cohort; and wherein determining the therapy timing to be used by applying the one or more mood scores to the mood-based personalization model includes applying each of the one or more enumerated mood scores to the mood-based personalization model.

Example 79 is the computer-implemented method of example(s) 78, wherein determining the therapy timing to be used by applying each of the one or more enumerated mood scores to the mood-based personalization model results in the determined therapy timing that is based at least in part on the intensity of each of the one or more unique moods.

Example 80 is the computer-implemented method of example(s) 74-79, wherein generating one or more mood scores includes: generating one or more enumerated mood scores, wherein each of the one or more enumerated mood scores is associated with a unique mood, and wherein each of the one or more enumerated mood scores is indicative of a strength of its respective unique mood; and generating an overall mood score based at least in part on the one or more enumerated mood scores.

Example 81 is the computer-implemented method of example(s) 74-80, wherein facilitating providing personalized therapy includes: determining a first time to initiate therapy based at least in part on the determined therapy timing; presenting a first prompt to begin therapy at the first time; determining a second time to initiate therapy based at least in part on the determined therapy timing; and presenting a second prompt to begin therapy at the second time.

Example 82 is the computer-implemented method of example(s) 74-81, wherein receiving the user input data includes: establishing, via a network interface, a chat interface with a user device associated with the user; and receiving, via the chat interface, the user input data, wherein facilitating providing the personalized therapy includes initiating the therapy tool via the chat interface.

Example 83 is the computer-implemented method of example(s) 74-82, wherein the mood-based personalization model is further trained using additional training data associated with the user, wherein the additional training data includes historical mood information associated with historical therapy tool information, wherein the historical therapy tool information is indicative of a historical therapy timing of applying one or more therapy tools, and wherein the historical mood information is indicative of i) at least one historical mood score that occurred prior to the historical therapy timing; ii) at least one historical mood score that occurred during the historical therapy timing; iii) at least one historical mood score that occurred after the historical therapy timing; or iv) any combination of i to iii.

Example 84 is a computer-program product tangibly embodied in a non-transitory machine-readable storage medium, including instructions configured to cause a data processing apparatus to perform operations including: receiving cohort information associated with a user; receiving mood information associated with the user, wherein receiving the mood information includes receiving user input data and generating one or more mood scores based at least in part on the input data; accessing a mood-based personalization model based on the cohort information, wherein the mood-based personalization model is trained, at least in part, using training data for a plurality individuals associated with the cohort, wherein the training data includes a plurality of mood scores associated with the plurality of individuals associated with the cohort; determining therapy timing to be used by applying the one or more mood scores to the mood-based personalization model, wherein the determined therapy timing is indicative of i) a frequency for applying one or more therapy tools; ii) a future time to apply the one or more therapy tools; or iii) a combination of i and ii; and facilitating providing personalized therapy to the user using the determined therapy timing.

Example 85 is the computer-program product of example(s) 84, wherein determining the therapy timing includes: selecting a therapy tool to use out of the one or more therapy tools; and determining the frequency for applying the therapy tool by applying the one or more mood scores and the selected therapy tool to the mood-based personalization model, wherein the plurality of mood scores of the training data includes a set of mood scores that are associated with the therapy tool.

Example 86 is the computer-program product of example(s) 84 or 85, wherein receiving cohort information associated with the user includes: receiving additional user input data; and assigning the user to the cohort based at least in part on the additional user input data.

Example 87 is the computer-program product of example(s) 84-86, wherein generating one or more mood scores includes generating an overall mood score, wherein the overall mood score is indicative of a positive mood or a negative mood, and wherein the overall mood score is further indicative of a strength of the positive mood or the negative mood.

Example 88 is the computer-program product of example(s) 84-87, wherein generating one or more mood scores includes generating one or more enumerated mood scores, wherein each of the one or more enumerated mood scores is associated with a unique mood, and wherein each of the one or more enumerated mood scores is indicative of a strength of its respective unique mood; wherein the plurality of mood scores associated with the plurality of individuals associated with the cohort includes a plurality of enumerated mood scores associated with the plurality of individuals associated with the cohort; and wherein determining the therapy timing to be used by applying the one or more mood scores to the mood-based personalization model includes applying each of the one or more enumerated mood scores to the mood-based personalization model.

Example 89 is the computer-program product of example(s) 88, wherein determining the therapy timing to be used by applying each of the one or more enumerated mood scores to the mood-based personalization model results in the determined therapy timing that is based at least in part on the intensity of each of the one or more unique moods.

Example 90 is the computer-program product of example(s) 84-89, wherein generating one or more mood scores includes: generating one or more enumerated mood scores, wherein each of the one or more enumerated mood scores is associated with a unique mood, and wherein each of the one or more enumerated mood scores is indicative of a strength of its respective unique mood; and generating an overall mood score based at least in part on the one or more enumerated mood scores.

Example 91 is the computer-program product of example(s) 84-90, wherein facilitating providing personalized therapy includes: determining a first time to initiate therapy based at least in part on the determined therapy timing; presenting a first prompt to begin therapy at the first time; determining a second time to initiate therapy based at least in part on the determined therapy timing; and presenting a second prompt to begin therapy at the second time.

Example 92 is the computer-program product of example(s) 84-91, wherein receiving the user input data includes: establishing, via a network interface, a chat interface with a user device associated with the user; and receiving, via the chat interface, the user input data, wherein facilitating providing the personalized therapy includes initiating the therapy tool via the chat interface.

Example 93 is the computer-program product of example(s) 84-92, wherein the mood-based personalization model is further trained using additional training data associated with the user, wherein the additional training data includes historical mood information associated with historical therapy tool information, wherein the historical therapy tool information is indicative of a historical therapy timing of applying one or more therapy tools, and wherein the historical mood information is indicative of i) at least one historical mood score that occurred prior to the historical therapy timing; ii) at least one historical mood score that occurred during the historical therapy timing; iii) at least one historical mood score that occurred after the historical therapy timing; or iv) any combination of i to iii.

Example 94 is a system, comprising: one or more data processors; and a non-transitory computer-readable storage medium containing instructions which, when executed on the one or more data processors, cause the one or more data processors to perform operations including: receiving first user input data associated with a therapy target; generating one or more first assessment scores based at least in part on the first user input data, wherein the one or more first assessment scores are indicative of i) a pre-therapy perceived severity of the therapy target; ii) a pre-therapy perceived severity of a condition associated with the therapy target; or iii) a combination of i and ii; providing, after receiving the first user input data, therapy to a user using a therapy tool during a therapy session; receiving second user input data associated with the therapy target, wherein receiving the second user input data occurs after providing the therapy; generating one or more second assessment scores based at least in part on the second user input data, wherein the one or more second assessment scores are indicative of i) a post-therapy perceived severity of the therapy target; ii) a post-therapy perceived severity of the condition associated with the therapy target; or iii) a combination of i and ii; training an assessment-based personalization model based at least in part on the one or more first assessment scores, the one or more second assessment scores, and the provided therapy; determining a subsequent therapy tool to be used during a subsequent therapy session associated with the therapy target, wherein determining the subsequent therapy tool is based at least in part on the trained assessment-based personalization model; and facilitating providing personalized therapy to the user using the determined therapy tool.

Example 95 is the system of example(s) 94, wherein determining the subsequent therapy tool includes selecting the subsequent therapy tool out of a plurality of possible therapy tools, wherein the plurality of possible therapy tools includes the therapy tool used during the therapy session.

Example 96 is the system of example(s) 94 or 95, wherein determining the therapy tool includes: generating a therapy tool effectiveness score for each of the plurality of possible therapy tools based at least in part on the assessment-based personalization model; ranking each of the plurality of possible therapy tools based on the respective therapy tool effectiveness score; and selecting one of the plurality of possible therapy tools based on the ranking.

Example 97 is the system of example(s) 94-96, wherein generating the one or more first assessment scores includes generating a first overall assessment score, wherein the first overall assessment score is indicative of an overall pre-therapy perceived severity of the therapy target, wherein generating the one or more second assessment scores includes generating a second overall assessment score, wherein the second overall assessment score is indicative of an overall post-therapy perceived severity of the therapy target.

Example 98 is the system of example(s) 94-97, wherein generating the first one or more assessment scores includes generating a plurality of first category assessment scores, wherein each of the plurality of first category assessment scores is associated with a unique category of problem associated with the therapy target, and wherein each of the plurality of first category assessment scores is indicative of a strength of its respective unique category of problem; wherein generating the second one or more assessment scores includes generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and wherein training the assessment-based personalization model is based at least in part on the plurality of first category assessment scores, the plurality of second assessment scores, and the provided therapy.

Example 99 is the system of example(s) 94-98, wherein generating the first one or more assessment scores includes generating a first overall assessment score, and wherein generating the first overall assessment score includes: generating a plurality of first category assessment scores, wherein each of the plurality of first category assessment scores is associated with a unique category of problem associated with the therapy target, and wherein each of the plurality of first category assessment scores is indicative of a strength of its respective unique category of problem; and calculating the first overall assessment score based at least in part on the plurality of first category assessment scores; and wherein generating the second one or more assessment scores includes generating a second overall assessment score, and wherein generating the second overall assessment score includes: generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and calculating the second overall assessment score based at least in part on the plurality of second category assessment scores.

Example 100 is the system of example(s) 94-99, wherein facilitating providing personalized therapy includes presenting a prompt to begin subsequent therapy using the determined subsequent therapy tool.

Example 101 is the system of example(s) 94-100 wherein receiving the first user input data includes: establishing, via a network interface, a chat interface with a user device associated with the user; and receiving, via the chat interface, the first user input data; wherein receiving the second user input data includes receiving, via the chat interface, the second user input data; and wherein facilitating providing the personalized therapy includes initiating the subsequent therapy tool via the chat interface.

Example 102 is the system of example(s) 94-101, wherein the assessment-based personalization model is further trained using training data, wherein the training data is associated with a plurality of historical therapy sessions, and wherein, for each of the plurality of historical therapy sessions, the training data includes: a historical first assessment score; a historical second assessment score; and a historical provided therapy associated with the historical therapy session.

Example 103 is the system of example(s) 94-102, wherein training the assessment-based personalization model includes accessing and further training an existing assessment-based personalization model, wherein the existing assessment-based personalization model is trained using training data associated with a cohort of users, the training data including pre-therapy assessment scores and post-therapy assessment scores associated with a plurality of historical provided therapy, and wherein the user is a member of the cohort of users.

Example 104 is the system of example(s) 94-103, wherein the operations further include receiving mood information associated with the user, wherein receiving the mood information includes receiving subsequent user input data and generating one or more mood scores based at least in part on the subsequent input data, wherein determining the subsequent therapy tool is further based on applying the one or more mood scores to a mood-based personalization model, wherein the mood-based personalization model is trained, at least in part, using training data that includes a plurality of historical mood scores associated with a plurality of historical therapy sessions provided to the user.

Example 105 is a computer-implemented method, comprising: receiving first user input data associated with a therapy target; generating one or more first assessment scores based at least in part on the first user input data, wherein the one or more first assessment scores are indicative of i) a pre-therapy perceived severity of the therapy target; ii) a pre-therapy perceived severity of a condition associated with the therapy target; or iii) a combination of i and ii; providing, after receiving the first user input data, therapy to a user using a therapy tool during a therapy session; receiving second user input data associated with the therapy target, wherein receiving the second user input data occurs after providing the therapy; generating one or more second assessment scores based at least in part on the second user input data, wherein the one or more second assessment scores are indicative of i) a post-therapy perceived severity of the therapy target; ii) a post-therapy perceived severity of the condition associated with the therapy target; or iii) a combination of i and ii; training an assessment-based personalization model based at least in part on the one or more first assessment scores, the one or more second assessment scores, and the provided therapy; determining a subsequent therapy tool to be used during a subsequent therapy session associated with the therapy target, wherein determining the subsequent therapy tool is based at least in part on the trained assessment-based personalization model; and facilitating providing personalized therapy to the user using the determined therapy tool.

Example 106 is the computer-implemented method of example(s) 105, wherein determining the subsequent therapy tool includes selecting the subsequent therapy tool out of a plurality of possible therapy tools, wherein the plurality of possible therapy tools includes the therapy tool used during the therapy session.

Example 107 is the computer-implemented method of example(s) 105 or 106, wherein determining the therapy tool includes: generating a therapy tool effectiveness score for each of the plurality of possible therapy tools based at least in part on the assessment-based personalization model; ranking each of the plurality of possible therapy tools based on the respective therapy tool effectiveness score; and selecting one of the plurality of possible therapy tools based on the ranking.

Example 108 is the computer-implemented method of example(s) 105-107, wherein generating the one or more first assessment scores includes generating a first overall assessment score, wherein the first overall assessment score is indicative of an overall pre-therapy perceived severity of the therapy target, wherein generating the one or more second assessment scores includes generating a second overall assessment score, wherein the second overall assessment score is indicative of an overall post-therapy perceived severity of the therapy target.

Example 109 is the computer-implemented method of example(s) 105-108, wherein generating the first one or more assessment scores includes generating a plurality of first category assessment scores, wherein each of the plurality of first category assessment scores is associated with a unique category of problem associated with the therapy target, and wherein each of the plurality of first category assessment scores is indicative of a strength of its respective unique category of problem; wherein generating the second one or more assessment scores includes generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and wherein training the assessment-based personalization model is based at least in part on the plurality of first category assessment scores, the plurality of second assessment scores, and the provided therapy.

Example 110 is the computer-implemented method of example(s) 105-109, wherein generating the first one or more assessment scores includes generating a first overall assessment score, and wherein generating the first overall assessment score includes: generating a plurality of first category assessment scores, wherein each of the plurality of first category assessment scores is associated with a unique category of problem associated with the therapy target, and wherein each of the plurality of first category assessment scores is indicative of a strength of its respective unique category of problem; and calculating the first overall assessment score based at least in part on the plurality of first category assessment scores; and wherein generating the second one or more assessment scores includes generating a second overall assessment score, and wherein generating the second overall assessment score includes: generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and calculating the second overall assessment score based at least in part on the plurality of second category assessment scores.

Example 111 is the computer-implemented method of example(s) 105-110, wherein facilitating providing personalized therapy includes presenting a prompt to begin subsequent therapy using the determined subsequent therapy tool.

Example 112 is the computer-implemented method of example(s) 105-111, wherein receiving the first user input data includes: establishing, via a network interface, a chat interface with a user device associated with the user; and receiving, via the chat interface, the first user input data; wherein receiving the second user input data includes receiving, via the chat interface, the second user input data; and wherein facilitating providing the personalized therapy includes initiating the subsequent therapy tool via the chat interface.

Example 113 is the computer-implemented method of example(s) 105-112, wherein the assessment-based personalization model is further trained using training data, wherein the training data is associated with a plurality of historical therapy sessions, and wherein, for each of the plurality of historical therapy sessions, the training data includes: a historical first assessment score; a historical second assessment score; and a historical provided therapy associated with the historical therapy session.

Example 114 is the computer-implemented method of example(s) 105-113, wherein training the assessment-based personalization model includes accessing and further training an existing assessment-based personalization model, wherein the existing assessment-based personalization model is trained using training data associated with a cohort of users, the training data including pre-therapy assessment scores and post-therapy assessment scores associated with a plurality of historical provided therapy, and wherein the user is a member of the cohort of users.

Example 115 is the computer-implemented method of example(s) 105-114, further comprising receiving mood information associated with the user, wherein receiving the mood information includes receiving subsequent user input data and generating one or more mood scores based at least in part on the subsequent input data, wherein determining the subsequent therapy tool is further based on applying the one or more mood scores to a mood-based personalization model, wherein the mood-based personalization model is trained, at least in part, using training data that includes a plurality of historical mood scores associated with a plurality of historical therapy sessions provided to the user.

Example 116 is a computer-program product tangibly embodied in a non-transitory machine-readable storage medium, including instructions configured to cause a data processing apparatus to perform operations including: receiving first user input data associated with a therapy target; generating one or more first assessment scores based at least in part on the first user input data, wherein the one or more first assessment scores are indicative of i) a pre-therapy perceived severity of the therapy target; ii) a pre-therapy perceived severity of a condition associated with the therapy target; or iii) a combination of i and ii; providing, after receiving the first user input data, therapy to a user using a therapy tool during a therapy session; receiving second user input data associated with the therapy target, wherein receiving the second user input data occurs after providing the therapy; generating one or more second assessment scores based at least in part on the second user input data, wherein the one or more second assessment scores are indicative of i) a post-therapy perceived severity of the therapy target; ii) a post-therapy perceived severity of the condition associated with the therapy target; or iii) a combination of i and ii; training an assessment-based personalization model based at least in part on the one or more first assessment scores, the one or more second assessment scores, and the provided therapy; determining a subsequent therapy tool to be used during a subsequent therapy session associated with the therapy target, wherein determining the subsequent therapy tool is based at least in part on the trained assessment-based personalization model; and facilitating providing personalized therapy to the user using the determined therapy tool.

Example 117 is the computer-program product of example(s) 116, wherein determining the subsequent therapy tool includes selecting the subsequent therapy tool out of a plurality of possible therapy tools, wherein the plurality of possible therapy tools includes the therapy tool used during the therapy session.

Example 118 is the computer-program product of example(s) 116 or 117, wherein determining the therapy tool includes: generating a therapy tool effectiveness score for each of the plurality of possible therapy tools based at least in part on the assessment-based personalization model; ranking each of the plurality of possible therapy tools based on the respective therapy tool effectiveness score; and selecting one of the plurality of possible therapy tools based on the ranking.

Example 119 is the computer-program product of example(s) 116-118, wherein generating the one or more first assessment scores includes generating a first overall assessment score, wherein the first overall assessment score is indicative of an overall pre-therapy perceived severity of the therapy target, wherein generating the one or more second assessment scores includes generating a second overall assessment score, wherein the second overall assessment score is indicative of an overall post-therapy perceived severity of the therapy target.

Example 120 is the computer-program product of example(s) 116-119, wherein generating the first one or more assessment scores includes generating a plurality of first category assessment scores, wherein each of the plurality of first category assessment scores is associated with a unique category of problem associated with the therapy target, and wherein each of the plurality of first category assessment scores is indicative of a strength of its respective unique category of problem; wherein generating the second one or more assessment scores includes generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and wherein training the assessment-based personalization model is based at least in part on the plurality of first category assessment scores, the plurality of second assessment scores, and the provided therapy.

Example 121 is the computer-program product of example(s) 116-120, wherein generating the first one or more assessment scores includes generating a first overall assessment score, and wherein generating the first overall assessment score includes: generating a plurality of first category assessment scores, wherein each of the plurality of first category assessment scores is associated with a unique category of problem associated with the therapy target, and wherein each of the plurality of first category assessment scores is indicative of a strength of its respective unique category of problem; and calculating the first overall assessment score based at least in part on the plurality of first category assessment scores; and wherein generating the second one or more assessment scores includes generating a second overall assessment score, and wherein generating the second overall assessment score includes: generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and calculating the second overall assessment score based at least in part on the plurality of second category assessment scores.

Example 122 is the computer-program product of example(s) 116-121, wherein facilitating providing personalized therapy includes presenting a prompt to begin subsequent therapy using the determined subsequent therapy tool.

Example 123 is the computer-program product of example(s) 116-122, wherein receiving the first user input data includes: establishing, via a network interface, a chat interface with a user device associated with the user; and receiving, via the chat interface, the first user input data; wherein receiving the second user input data includes receiving, via the chat interface, the second user input data; and wherein facilitating providing the personalized therapy includes initiating the subsequent therapy tool via the chat interface.

Example 124 is the computer-program product of example(s) 116-123, wherein the assessment-based personalization model is further trained using training data, wherein the training data is associated with a plurality of historical therapy sessions, and wherein, for each of the plurality of historical therapy sessions, the training data includes: a historical first assessment score; a historical second assessment score; and a historical provided therapy associated with the historical therapy session.

Example 125 is the computer-program product of example(s) 116-124, wherein training the assessment-based personalization model includes accessing and further training an existing assessment-based personalization model, wherein the existing assessment-based personalization model is trained using training data associated with a cohort of users, the training data including pre-therapy assessment scores and post-therapy assessment scores associated with a plurality of historical provided therapy, and wherein the user is a member of the cohort of users.

Example 126 is the computer-program product of example(s) 116-125, wherein the operations further include receiving mood information associated with the user, wherein receiving the mood information includes receiving subsequent user input data and generating one or more mood scores based at least in part on the subsequent input data, wherein determining the subsequent therapy tool is further based on applying the one or more mood scores to a mood-based personalization model, wherein the mood-based personalization model is trained, at least in part, using training data that includes a plurality of historical mood scores associated with a plurality of historical therapy sessions provided to the user.

Example 127 is a system, comprising: one or more data processors; and a non-transitory computer-readable storage medium containing instructions which, when executed on the one or more data processors, cause the one or more data processors to perform operations including: receiving first user input data associated with a therapy target; generating one or more first assessment scores based at least in part on the first user input data, wherein the one or more first assessment scores are indicative of i) a pre-therapy perceived severity of the therapy target; ii) a pre-therapy perceived severity of a condition associated with the therapy target; or iii) a combination of i and ii; providing, after receiving the first user input data, therapy to a user using a therapy tool during a therapy session; receiving second user input data associated with the therapy target, wherein receiving the second user input data occurs after providing the therapy; generating one or more second assessment scores based at least in part on the second user input data, wherein the one or more second assessment scores are indicative of i) a post-therapy perceived severity of the therapy target; ii) a post-therapy perceived severity of the condition associated with the therapy target; or iii) a combination of i and ii; training an assessment-based personalization model based at least in part on the one or more first assessment scores, the one or more second assessment scores, and the provided therapy; determining therapy sequencing to be used during one or more subsequent therapy sessions associated with the therapy target, wherein determining the therapy sequencing is based at least in part on the trained assessment-based personalization model, and wherein the determined therapy sequencing is indicative of i) an order for applying a plurality of therapy tools; ii) an order for addressing a plurality of therapy targets including the therapy target; or iii) a combination of i and ii; and facilitating providing personalized therapy to the user using the determined therapy sequencing.

Example 128 is the system of example(s) 127, wherein determining the therapy sequencing includes: receiving subsequent user input data, wherein the subsequent user input data is indicative of a subsequent therapy target to be treated with the one or more subsequent therapy sessions; identifying a set of related therapy targets that are related to the subsequent therapy target; and generating an order for addressing the set of related therapy targets and the subsequent therapy target based at least in part on the identified set of related therapy targets, the subsequent therapy target, and the trained assessment-based personalization model.

Example 129 is the system of example(s) 128, wherein generating an order for addressing the set of related therapy targets and the subsequent therapy target include: generating, for each of the identified set of related therapy targets and the subsequent therapy target, an effectiveness score using the trained assessment-based personalization model; ranking each of the identified set of related therapy targets and the subsequent therapy target according to its respective effectiveness score; and selecting the order based at least in part on the ranking.

Example 130 is the system of example(s) 127-129, wherein generating the one or more first assessment scores includes generating a first overall assessment score, wherein the first overall assessment score is indicative of an overall pre-therapy perceived severity of the therapy target, wherein generating the one or more second assessment scores includes generating a second overall assessment score, wherein the second overall assessment score is indicative of an overall post-therapy perceived severity of the therapy target.

Example 131 is the system of example(s) 127-130, wherein generating the first one or more assessment scores includes generating a plurality of first category assessment scores, wherein each of the plurality of first category assessment scores is associated with a unique category of problem associated with the therapy target, and wherein each of the plurality of first category assessment scores is indicative of a strength of its respective unique category of problem; wherein generating the second one or more assessment scores includes generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and wherein training the assessment-based personalization model is based at least in part on the plurality of first category assessment scores, the plurality of second assessment scores, and the provided therapy.

Example 132 is the system of example(s) 127-131, wherein generating the first one or more assessment scores includes generating a first overall assessment score, and wherein generating the first overall assessment score includes: generating a plurality of first category assessment scores, wherein each of the plurality of first category assessment scores is associated with a unique category of problem associated with the therapy target, and wherein each of the plurality of first category assessment scores is indicative of a strength of its respective unique category of problem; and calculating the first overall assessment score based at least in part on the plurality of first category assessment scores; and wherein generating the second one or more assessment scores includes generating a second overall assessment score, and wherein generating the second overall assessment score includes: generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and calculating the second overall assessment score based at least in part on the plurality of second category assessment scores.

Example 133 is the system of example(s) 127-132, wherein facilitating providing personalized therapy includes presenting a prompt to begin subsequent therapy using the determined therapy sequencing.

Example 134 is the system of example(s) 127-133, wherein receiving the first user input data includes: establishing, via a network interface, a chat interface with a user device associated with the user; and receiving, via the chat interface, the first user input data; wherein receiving the second user input data includes receiving, via the chat interface, the second user input data; and wherein facilitating providing the personalized therapy includes initiating subsequent therapy, via the chat interface, using the determined therapy sequencing.

Example 135 is the system of example(s) 127-134, wherein the assessment-based personalization model is further trained using training data, wherein the training data is associated with a plurality of historical therapy sessions, and wherein, for each of the plurality of historical therapy sessions, the training data includes: a historical first assessment score; a historical second assessment score; and a historical provided therapy associated with the historical therapy session.

Example 136 is the system of example(s) 127-135, wherein training the assessment-based personalization model includes accessing and further training an existing assessment-based personalization model, wherein the existing assessment-based personalization model is trained using training data associated with a cohort of users, the training data including pre-therapy assessment scores and post-therapy assessment scores associated with a plurality of historical provided therapy, and wherein the user is a member of the cohort of users.

Example 137 is the system of example(s) 127-136, wherein the operations further include receiving mood information associated with the user, wherein receiving the mood information includes receiving subsequent user input data and generating one or more mood scores based at least in part on the subsequent input data, wherein determining the therapy sequencing is further based on applying the one or more mood scores to a mood-based personalization model, wherein the mood-based personalization model is trained, at least in part, using training data that includes a plurality of historical mood scores associated with a plurality of historical therapy sessions provided to the user.

Example 138 is a computer-implemented method, comprising: receiving first user input data associated with a therapy target; generating one or more first assessment scores based at least in part on the first user input data, wherein the one or more first assessment scores are indicative of i) a pre-therapy perceived severity of the therapy target; ii) a pre-therapy perceived severity of a condition associated with the therapy target; or iii) a combination of i and ii; providing, after receiving the first user input data, therapy to a user using a therapy tool during a therapy session; receiving second user input data associated with the therapy target, wherein receiving the second user input data occurs after providing the therapy; generating one or more second assessment scores based at least in part on the second user input data, wherein the one or more second assessment scores are indicative of i) a post-therapy perceived severity of the therapy target; ii) a post-therapy perceived severity of the condition associated with the therapy target; or iii) a combination of i and ii; training an assessment-based personalization model based at least in part on the one or more first assessment scores, the one or more second assessment scores, and the provided therapy; determining therapy sequencing to be used during one or more subsequent therapy sessions associated with the therapy target, wherein determining the therapy sequencing is based at least in part on the trained assessment-based personalization model, and wherein the determined therapy sequencing is indicative of i) an order for applying a plurality of therapy tools; ii) an order for addressing a plurality of therapy targets including the therapy target; or iii) a combination of i and ii; and facilitating providing personalized therapy to the user using the determined therapy sequencing.

Example 139 is the computer-implemented method of example(s) 138, wherein determining the therapy sequencing includes: receiving subsequent user input data, wherein the subsequent user input data is indicative of a subsequent therapy target to be treated with the one or more subsequent therapy sessions; identifying a set of related therapy targets that are related to the subsequent therapy target; and generating an order for addressing the set of related therapy targets and the subsequent therapy target based at least in part on the identified set of related therapy targets, the subsequent therapy target, and the trained assessment-based personalization model.

Example 140 is the computer-implemented method of example(s) 139, wherein generating an order for addressing the set of related therapy targets and the subsequent therapy target include: generating, for each of the identified set of related therapy targets and the subsequent therapy target, an effectiveness score using the trained assessment-based personalization model; ranking each of the identified set of related therapy targets and the subsequent therapy target according to its respective effectiveness score; and selecting the order based at least in part on the ranking.

Example 141 is the computer-implemented method of example(s) 138-140, wherein generating the one or more first assessment scores includes generating a first overall assessment score, wherein the first overall assessment score is indicative of an overall pre-therapy perceived severity of the therapy target, wherein generating the one or more second assessment scores includes generating a second overall assessment score, wherein the second overall assessment score is indicative of an overall post-therapy perceived severity of the therapy target.

Example 142 is the computer-implemented method of example(s) 138-141 wherein generating the first one or more assessment scores includes generating a plurality of first category assessment scores, wherein each of the plurality of first category assessment scores is associated with a unique category of problem associated with the therapy target, and wherein each of the plurality of first category assessment scores is indicative of a strength of its respective unique category of problem; wherein generating the second one or more assessment scores includes generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and wherein training the assessment-based personalization model is based at least in part on the plurality of first category assessment scores, the plurality of second assessment scores, and the provided therapy.

Example 143 is the computer-implemented method of example(s) 138-142, wherein generating the first one or more assessment scores includes generating a first overall assessment score, and wherein generating the first overall assessment score includes: generating a plurality of first category assessment scores, wherein each of the plurality of first category assessment scores is associated with a unique category of problem associated with the therapy target, and wherein each of the plurality of first category assessment scores is indicative of a strength of its respective unique category of problem; and calculating the first overall assessment score based at least in part on the plurality of first category assessment scores; and wherein generating the second one or more assessment scores includes generating a second overall assessment score, and wherein generating the second overall assessment score includes: generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and calculating the second overall assessment score based at least in part on the plurality of second category assessment scores.

Example 144 is the computer-implemented method of example(s) 138-143, wherein facilitating providing personalized therapy includes presenting a prompt to begin subsequent therapy using the determined therapy sequencing.

Example 145 is the computer-implemented method of example(s) 138-144, wherein receiving the first user input data includes: establishing, via a network interface, a chat interface with a user device associated with the user; and receiving, via the chat interface, the first user input data; wherein receiving the second user input data includes receiving, via the chat interface, the second user input data; and wherein facilitating providing the personalized therapy includes initiating subsequent therapy, via the chat interface, using the determined therapy sequencing.

Example 146 is the computer-implemented method of example(s) 138-145, wherein the assessment-based personalization model is further trained using training data, wherein the training data is associated with a plurality of historical therapy sessions, and wherein, for each of the plurality of historical therapy sessions, the training data includes: a historical first assessment score; a historical second assessment score; and a historical provided therapy associated with the historical therapy session.

Example 147 is the computer-implemented method of example(s) 138-146, wherein training the assessment-based personalization model includes accessing and further training an existing assessment-based personalization model, wherein the existing assessment-based personalization model is trained using training data associated with a cohort of users, the training data including pre-therapy assessment scores and post-therapy assessment scores associated with a plurality of historical provided therapy, and wherein the user is a member of the cohort of users.

Example 148 is the computer-implemented method of example(s) 138-147, wherein the operations further include receiving mood information associated with the user, wherein receiving the mood information includes receiving subsequent user input data and generating one or more mood scores based at least in part on the subsequent input data, wherein determining the therapy sequencing is further based on applying the one or more mood scores to a mood-based personalization model, wherein the mood-based personalization model is trained, at least in part, using training data that includes a plurality of historical mood scores associated with a plurality of historical therapy sessions provided to the user.

Example 149 is a computer-program product tangibly embodied in a non-transitory machine-readable storage medium, including instructions configured to cause a data processing apparatus to perform operations including: receiving first user input data associated with a therapy target; generating one or more first assessment scores based at least in part on the first user input data, wherein the one or more first assessment scores are indicative of i) a pre-therapy perceived severity of the therapy target; ii) a pre-therapy perceived severity of a condition associated with the therapy target; or iii) a combination of i and ii; providing, after receiving the first user input data, therapy to a user using a therapy tool during a therapy session; receiving second user input data associated with the therapy target, wherein receiving the second user input data occurs after providing the therapy; generating one or more second assessment scores based at least in part on the second user input data, wherein the one or more second assessment scores are indicative of i) a post-therapy perceived severity of the therapy target; ii) a post-therapy perceived severity of the condition associated with the therapy target; or iii) a combination of i and ii; training an assessment-based personalization model based at least in part on the one or more first assessment scores, the one or more second assessment scores, and the provided therapy; determining therapy sequencing to be used during one or more subsequent therapy sessions associated with the therapy target, wherein determining the therapy sequencing is based at least in part on the trained assessment-based personalization model, and wherein the determined therapy sequencing is indicative of i) an order for applying a plurality of therapy tools; ii) an order for addressing a plurality of therapy targets including the therapy target; or iii) a combination of i and ii; and facilitating providing personalized therapy to the user using the determined therapy sequencing.

Example 150 is the computer-program product of example(s) 149, wherein determining the therapy sequencing includes: receiving subsequent user input data, wherein the subsequent user input data is indicative of a subsequent therapy target to be treated with the one or more subsequent therapy sessions; identifying a set of related therapy targets that are related to the subsequent therapy target; and generating an order for addressing the set of related therapy targets and the subsequent therapy target based at least in part on the identified set of related therapy targets, the subsequent therapy target, and the trained assessment-based personalization model.

Example 151 is the computer-program product of example(s) 150, wherein generating an order for addressing the set of related therapy targets and the subsequent therapy target include: generating, for each of the identified set of related therapy targets and the subsequent therapy target, an effectiveness score using the trained assessment-based personalization model; ranking each of the identified set of related therapy targets and the subsequent therapy target according to its respective effectiveness score; and selecting the order based at least in part on the ranking.

Example 152 is the computer-program product of example(s) 149-151, wherein generating the one or more first assessment scores includes generating a first overall assessment score, wherein the first overall assessment score is indicative of an overall pre-therapy perceived severity of the therapy target, wherein generating the one or more second assessment scores includes generating a second overall assessment score, wherein the second overall assessment score is indicative of an overall post-therapy perceived severity of the therapy target.

Example 153 is the computer-program product of example(s) 149-152, wherein generating the first one or more assessment scores includes generating a plurality of first category assessment scores, wherein each of the plurality of first category assessment scores is associated with a unique category of problem associated with the therapy target, and wherein each of the plurality of first category assessment scores is indicative of a strength of its respective unique category of problem; wherein generating the second one or more assessment scores includes generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and wherein training the assessment-based personalization model is based at least in part on the plurality of first category assessment scores, the plurality of second assessment scores, and the provided therapy.

Example 154 is the computer-program product of example(s) 149-153, wherein generating the first one or more assessment scores includes generating a first overall assessment score, and wherein generating the first overall assessment score includes: generating a plurality of first category assessment scores, wherein each of the plurality of first category assessment scores is associated with a unique category of problem associated with the therapy target, and wherein each of the plurality of first category assessment scores is indicative of a strength of its respective unique category of problem; and calculating the first overall assessment score based at least in part on the plurality of first category assessment scores; and wherein generating the second one or more assessment scores includes generating a second overall assessment score, and wherein generating the second overall assessment score includes: generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and calculating the second overall assessment score based at least in part on the plurality of second category assessment scores.

Example 155 is the computer-program product of example(s) 149-154, wherein facilitating providing personalized therapy includes presenting a prompt to begin subsequent therapy using the determined therapy sequencing.

Example 156 is the computer-program product of example(s) 149-155, wherein receiving the first user input data includes: establishing, via a network interface, a chat interface with a user device associated with the user; and receiving, via the chat interface, the first user input data; wherein receiving the second user input data includes receiving, via the chat interface, the second user input data; and wherein facilitating providing the personalized therapy includes initiating subsequent therapy, via the chat interface, using the determined therapy sequencing.

Example 157 is the computer-program product of example(s) 149-156, wherein the assessment-based personalization model is further trained using training data, wherein the training data is associated with a plurality of historical therapy sessions, and wherein, for each of the plurality of historical therapy sessions, the training data includes: a historical first assessment score; a historical second assessment score; and a historical provided therapy associated with the historical therapy session.

Example 158 is the computer-program product of example(s) 149-157, wherein training the assessment-based personalization model includes accessing and further training an existing assessment-based personalization model, wherein the existing assessment-based personalization model is trained using training data associated with a cohort of users, the training data including pre-therapy assessment scores and post-therapy assessment scores associated with a plurality of historical provided therapy, and wherein the user is a member of the cohort of users.

Example 159 is the computer-program product of example(s) 149-158, wherein the operations further include receiving mood information associated with the user, wherein receiving the mood information includes receiving subsequent user input data and generating one or more mood scores based at least in part on the subsequent input data, wherein determining the therapy sequencing is further based on applying the one or more mood scores to a mood-based personalization model, wherein the mood-based personalization model is trained, at least in part, using training data that includes a plurality of historical mood scores associated with a plurality of historical therapy sessions provided to the user.

Example 160 is a system, comprising: one or more data processors; and a non-transitory computer-readable storage medium containing instructions which, when executed on the one or more data processors, cause the one or more data processors to perform operations including: receiving first user input data associated with a therapy target; generating one or more first assessment scores based at least in part on the first user input data, wherein the one or more first assessment scores are indicative of i) a pre-therapy perceived severity of the therapy target; ii) a pre-therapy perceived severity of a condition associated with the therapy target; or iii) a combination of i and ii; providing, after receiving the first user input data, therapy to a user using a therapy tool during a therapy session; receiving second user input data associated with the therapy target, wherein receiving the second user input data occurs after providing the therapy; generating one or more second assessment scores based at least in part on the second user input data, wherein the one or more second assessment scores are indicative of i) a post-therapy perceived severity of the therapy target; ii) a post-therapy perceived severity of the condition associated with the therapy target; or iii) a combination of i and ii; training an assessment-based personalization model based at least in part on the one or more first assessment scores, the one or more second assessment scores, and the provided therapy; determining therapy timing to be used for one or more subsequent therapy sessions associated with the therapy target, wherein determining the therapy timing is based at least in part on the trained assessment-based personalization model, and wherein the therapy timing is indicative of i) a frequency for applying one or more therapy tools; ii) a future time to apply the one or more therapy tools; or iii) a combination of i and ii; facilitating providing personalized therapy to the user using the determined therapy timing.

Example 161 is the system of example(s) 160, wherein determining the therapy timing includes: receiving subsequent user input data, wherein the subsequent user input data is indicative of a subsequent therapy target to be treated with the one or more subsequent therapy sessions; determining the frequency for applying the one or more therapy tools based at least in part on the subsequent therapy target and the trained assessment-based personalization model.

Example 162 is the system of example(s) 160 or 161, wherein determining the therapy timing includes: comparing the one or more first assessment scores and the one or more second assessment scores to identify one or more assessment score trends; and adjusting the frequency for applying the one or more therapy tools based at least in part on the one or more assessment score trends.

Example 163 is the system of example(s) 160-162, wherein generating the one or more first assessment scores includes generating a first overall assessment score, wherein the first overall assessment score is indicative of an overall pre-therapy perceived severity of the therapy target, wherein generating the one or more second assessment scores includes generating a second overall assessment score, wherein the second overall assessment score is indicative of an overall post-therapy perceived severity of the therapy target.

Example 164 is the system of example(s) 160-163, wherein generating the first one or more assessment scores includes generating a plurality of first category assessment scores, wherein each of the plurality of first category assessment scores is associated with a unique category of problem associated with the therapy target, and wherein each of the plurality of first category assessment scores is indicative of a strength of its respective unique category of problem; wherein generating the second one or more assessment scores includes generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and wherein training the assessment-based personalization model is based at least in part on the plurality of first category assessment scores, the plurality of second assessment scores, and the provided therapy.

Example 165 is the system of example(s) 160-164, wherein generating the first one or more assessment scores includes generating a first overall assessment score, and wherein generating the first overall assessment score includes: generating a plurality of first category assessment scores, wherein each of the plurality of first category assessment scores is associated with a unique category of problem associated with the therapy target, and wherein each of the plurality of first category assessment scores is indicative of a strength of its respective unique category of problem; and calculating the first overall assessment score based at least in part on the plurality of first category assessment scores; and wherein generating the second one or more assessment scores includes generating a second overall assessment score, and wherein generating the second overall assessment score includes: generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and calculating the second overall assessment score based at least in part on the plurality of second category assessment scores.

Example 166 is the system of example(s) 160-165, wherein facilitating providing personalized therapy includes presenting a prompt to begin subsequent therapy using the determined therapy timing.

Example 167 is the system of example(s) 160-166 wherein receiving the first user input data includes: establishing, via a network interface, a chat interface with a user device associated with the user; and receiving, via the chat interface, the first user input data; wherein receiving the second user input data includes receiving, via the chat interface, the second user input data; and wherein facilitating providing the personalized therapy includes initiating subsequent therapy, via the chat interface, using the determined therapy timing.

Example 168 is the system of example(s) 160-167, wherein the assessment-based personalization model is further trained using training data, wherein the training data is associated with a plurality of historical therapy sessions, and wherein, for each of the plurality of historical therapy sessions, the training data includes: a historical first assessment score; a historical second assessment score; and a historical provided therapy associated with the historical therapy session.

Example 169 is the system of example(s) 160-168, wherein training the assessment-based personalization model includes accessing and further training an existing assessment-based personalization model, wherein the existing assessment-based personalization model is trained using training data associated with a cohort of users, the training data including pre-therapy assessment scores and post-therapy assessment scores associated with a plurality of historical provided therapy, and wherein the user is a member of the cohort of users.

Example 170 is the system of example(s) 160-169, wherein the operations further include receiving mood information associated with the user, wherein receiving the mood information includes receiving subsequent user input data and generating one or more mood scores based at least in part on the subsequent input data, wherein determining the therapy timing is further based on applying the one or more mood scores to a mood-based personalization model, wherein the mood-based personalization model is trained, at least in part, using training data that includes a plurality of historical mood scores associated with a plurality of historical therapy sessions provided to the user.

Example 171 is a computer-implemented method, comprising: receiving first user input data associated with a therapy target; generating one or more first assessment scores based at least in part on the first user input data, wherein the one or more first assessment scores are indicative of i) a pre-therapy perceived severity of the therapy target; ii) a pre-therapy perceived severity of a condition associated with the therapy target; or iii) a combination of i and ii; providing, after receiving the first user input data, therapy to a user using a therapy tool during a therapy session; receiving second user input data associated with the therapy target, wherein receiving the second user input data occurs after providing the therapy; generating one or more second assessment scores based at least in part on the second user input data, wherein the one or more second assessment scores are indicative of i) a post-therapy perceived severity of the therapy target; ii) a post-therapy perceived severity of the condition associated with the therapy target; or iii) a combination of i and ii; training an assessment-based personalization model based at least in part on the one or more first assessment scores, the one or more second assessment scores, and the provided therapy; determining therapy timing to be used for one or more subsequent therapy sessions associated with the therapy target, wherein determining the therapy timing is based at least in part on the trained assessment-based personalization model, and wherein the therapy timing is indicative of i) a frequency for applying one or more therapy tools; ii) a future time to apply the one or more therapy tools; or iii) a combination of i and ii; facilitating providing personalized therapy to the user using the determined therapy timing.

Example 172 is the computer-implemented method of example(s) 171, wherein determining the therapy timing includes: receiving subsequent user input data, wherein the subsequent user input data is indicative of a subsequent therapy target to be treated with the one or more subsequent therapy sessions; determining the frequency for applying the one or more therapy tools based at least in part on the subsequent therapy target and the trained assessment-based personalization model.

Example 173 is the computer-implemented method of example(s) 171 or 172, wherein determining the therapy timing includes: comparing the one or more first assessment scores and the one or more second assessment scores to identify one or more assessment score trends; and adjusting the frequency for applying the one or more therapy tools based at least in part on the one or more assessment score trends.

Example 174 is the computer-implemented method of example(s) 171-173, wherein generating the one or more first assessment scores includes generating a first overall assessment score, wherein the first overall assessment score is indicative of an overall pre-therapy perceived severity of the therapy target, wherein generating the one or more second assessment scores includes generating a second overall assessment score, wherein the second overall assessment score is indicative of an overall post-therapy perceived severity of the therapy target.

Example 175 is the computer-implemented method of example(s) 171-174, wherein generating the first one or more assessment scores includes generating a plurality of first category assessment scores, wherein each of the plurality of first category assessment scores is associated with a unique category of problem associated with the therapy target, and wherein each of the plurality of first category assessment scores is indicative of a strength of its respective unique category of problem; wherein generating the second one or more assessment scores includes generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and wherein training the assessment-based personalization model is based at least in part on the plurality of first category assessment scores, the plurality of second assessment scores, and the provided therapy.

Example 176 is the computer-implemented method of example(s) 171-175, wherein generating the first one or more assessment scores includes generating a first overall assessment score, and wherein generating the first overall assessment score includes: generating a plurality of first category assessment scores, wherein each of the plurality of first category assessment scores is associated with a unique category of problem associated with the therapy target, and wherein each of the plurality of first category assessment scores is indicative of a strength of its respective unique category of problem; and calculating the first overall assessment score based at least in part on the plurality of first category assessment scores; and wherein generating the second one or more assessment scores includes generating a second overall assessment score, and wherein generating the second overall assessment score includes: generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and calculating the second overall assessment score based at least in part on the plurality of second category assessment scores.

Example 177 is the computer-implemented method of example(s) 171-176, wherein facilitating providing personalized therapy includes presenting a prompt to begin subsequent therapy using the determined therapy timing.

Example 178 is the computer-implemented method of example(s) 171-177, wherein receiving the first user input data includes: establishing, via a network interface, a chat interface with a user device associated with the user; and receiving, via the chat interface, the first user input data; wherein receiving the second user input data includes receiving, via the chat interface, the second user input data; and wherein facilitating providing the personalized therapy includes initiating subsequent therapy, via the chat interface, using the determined therapy timing.

Example 179 is the computer-implemented method of example(s) 171-178, wherein the assessment-based personalization model is further trained using training data, wherein the training data is associated with a plurality of historical therapy sessions, and wherein, for each of the plurality of historical therapy sessions, the training data includes: a historical first assessment score; a historical second assessment score; and a historical provided therapy associated with the historical therapy session.

Example 180 is the computer-implemented method of example(s) 171-179, wherein training the assessment-based personalization model includes accessing and further training an existing assessment-based personalization model, wherein the existing assessment-based personalization model is trained using training data associated with a cohort of users, the training data including pre-therapy assessment scores and post-therapy assessment scores associated with a plurality of historical provided therapy, and wherein the user is a member of the cohort of users.

Example 181 is the computer-implemented method of example(s) 171-180, wherein the operations further include receiving mood information associated with the user, wherein receiving the mood information includes receiving subsequent user input data and generating one or more mood scores based at least in part on the subsequent input data, wherein determining the therapy timing is further based on applying the one or more mood scores to a mood-based personalization model, wherein the mood-based personalization model is trained, at least in part, using training data that includes a plurality of historical mood scores associated with a plurality of historical therapy sessions provided to the user.

Example 182 is a computer-program product tangibly embodied in a non-transitory machine-readable storage medium, including instructions configured to cause a data processing apparatus to perform operations including: receiving first user input data associated with a therapy target; generating one or more first assessment scores based at least in part on the first user input data, wherein the one or more first assessment scores are indicative of i) a pre-therapy perceived severity of the therapy target; ii) a pre-therapy perceived severity of a condition associated with the therapy target; or iii) a combination of i and ii; providing, after receiving the first user input data, therapy to a user using a therapy tool during a therapy session; receiving second user input data associated with the therapy target, wherein receiving the second user input data occurs after providing the therapy; generating one or more second assessment scores based at least in part on the second user input data, wherein the one or more second assessment scores are indicative of i) a post-therapy perceived severity of the therapy target; ii) a post-therapy perceived severity of the condition associated with the therapy target; or iii) a combination of i and ii; training an assessment-based personalization model based at least in part on the one or more first assessment scores, the one or more second assessment scores, and the provided therapy; determining therapy timing to be used for one or more subsequent therapy sessions associated with the therapy target, wherein determining the therapy timing is based at least in part on the trained assessment-based personalization model, and wherein the therapy timing is indicative of i) a frequency for applying one or more therapy tools; ii) a future time to apply the one or more therapy tools; or iii) a combination of i and ii; facilitating providing personalized therapy to the user using the determined therapy timing.

Example 183 is the computer-program product of example(s) 182, wherein determining the therapy timing includes: receiving subsequent user input data, wherein the subsequent user input data is indicative of a subsequent therapy target to be treated with the one or more subsequent therapy sessions; determining the frequency for applying the one or more therapy tools based at least in part on the subsequent therapy target and the trained assessment-based personalization model.

Example 184 is the computer-program product of example(s) 182 or 183, wherein determining the therapy timing includes: comparing the one or more first assessment scores and the one or more second assessment scores to identify one or more assessment score trends; and adjusting the frequency for applying the one or more therapy tools based at least in part on the one or more assessment score trends.

Example 185 is the computer-program product of example(s) 182-184, wherein generating the one or more first assessment scores includes generating a first overall assessment score, wherein the first overall assessment score is indicative of an overall pre-therapy perceived severity of the therapy target, wherein generating the one or more second assessment scores includes generating a second overall assessment score, wherein the second overall assessment score is indicative of an overall post-therapy perceived severity of the therapy target.

Example 186 is the computer-program product of example(s) 182-185, wherein generating the first one or more assessment scores includes generating a plurality of first category assessment scores, wherein each of the plurality of first category assessment scores is associated with a unique category of problem associated with the therapy target, and wherein each of the plurality of first category assessment scores is indicative of a strength of its respective unique category of problem; wherein generating the second one or more assessment scores includes generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and wherein training the assessment-based personalization model is based at least in part on the plurality of first category assessment scores, the plurality of second assessment scores, and the provided therapy.

Example 187 is the computer-program product of example(s) 182-186, wherein generating the first one or more assessment scores includes generating a first overall assessment score, and wherein generating the first overall assessment score includes: generating a plurality of first category assessment scores, wherein each of the plurality of first category assessment scores is associated with a unique category of problem associated with the therapy target, and wherein each of the plurality of first category assessment scores is indicative of a strength of its respective unique category of problem; and calculating the first overall assessment score based at least in part on the plurality of first category assessment scores; and wherein generating the second one or more assessment scores includes generating a second overall assessment score, and wherein generating the second overall assessment score includes: generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and calculating the second overall assessment score based at least in part on the plurality of second category assessment scores.

Example 188 is the computer-program product of example(s) 182-187, wherein facilitating providing personalized therapy includes presenting a prompt to begin subsequent therapy using the determined therapy timing.

Example 189 is the computer-program product of example(s) 182-188, wherein receiving the first user input data includes: establishing, via a network interface, a chat interface with a user device associated with the user; and receiving, via the chat interface, the first user input data; wherein receiving the second user input data includes receiving, via the chat interface, the second user input data; and wherein facilitating providing the personalized therapy includes initiating subsequent therapy, via the chat interface, using the determined therapy timing.

Example 190 is the computer-program product of example(s) 182-189, wherein the assessment-based personalization model is further trained using training data, wherein the training data is associated with a plurality of historical therapy sessions, and wherein, for each of the plurality of historical therapy sessions, the training data includes: a historical first assessment score; a historical second assessment score; and a historical provided therapy associated with the historical therapy session.

Example 191 is the computer-program product of example(s) 182-190, wherein training the assessment-based personalization model includes accessing and further training an existing assessment-based personalization model, wherein the existing assessment-based personalization model is trained using training data associated with a cohort of users, the training data including pre-therapy assessment scores and post-therapy assessment scores associated with a plurality of historical provided therapy, and wherein the user is a member of the cohort of users.

Example 192 is the computer-program product of example(s) 182-191, wherein the operations further include receiving mood information associated with the user, wherein receiving the mood information includes receiving subsequent user input data and generating one or more mood scores based at least in part on the subsequent input data, wherein determining the therapy timing is further based on applying the one or more mood scores to a mood-based personalization model, wherein the mood-based personalization model is trained, at least in part, using training data that includes a plurality of historical mood scores associated with a plurality of historical therapy sessions provided to the user.

Example 193 is the system, method, or computer-program product of example(s) 1-192, wherein facilitating providing the personalized therapy includes providing therapy to the user, and wherein providing the therapy to the user includes generating and transmitting a first message associated with the determined therapy tool.

## Claims

1. A computer-implemented method, comprising:
receiving first user input data associated with a therapy target;
generating one or more first assessment scores based at least in part on the first user input data, wherein the one or more first assessment scores are indicative of i) a pre-therapy perceived severity of the therapy target; ii) a pre-therapy perceived severity of a condition associated with the therapy target; or iii) a combination of i and ii;
providing, after receiving the first user input data, therapy to a user using a therapy tool during a therapy session;
receiving second user input data associated with the therapy target, wherein receiving the second user input data occurs after providing the therapy;
generating one or more second assessment scores based at least in part on the second user input data, wherein the one or more second assessment scores are indicative of i) a post-therapy perceived severity of the therapy target; ii) a post-therapy perceived severity of the condition associated with the therapy target; or iii) a combination of i and ii;
training an assessment-based personalization model based at least in part on the one or more first assessment scores, the one or more second assessment scores, and the provided therapy;
determining a subsequent therapy tool to be used during a subsequent therapy session associated with the therapy target, wherein determining the subsequent therapy tool is based at least in part on the trained assessment-based personalization model; and
facilitating providing personalized therapy to the user using the determined therapy tool.

2. The computer-implemented method of claim 1, wherein determining the subsequent therapy tool includes selecting the subsequent therapy tool out of a plurality of possible therapy tools, wherein the plurality of possible therapy tools includes the therapy tool used during the therapy session.

3. The computer-implemented method of claim 1, wherein determining the therapy tool includes:
generating a therapy tool effectiveness score for each of the plurality of possible therapy tools based at least in part on the assessment-based personalization model;
ranking each of the plurality of possible therapy tools based on the respective therapy tool effectiveness score; and
selecting one of the plurality of possible therapy tools based on the ranking.

4. The computer-implemented method of claim 1, wherein generating the one or more first assessment scores includes generating a first overall assessment score, wherein the first overall assessment score is indicative of an overall pre-therapy perceived severity of the therapy target, wherein generating the one or more second assessment scores includes generating a second overall assessment score, wherein the second overall assessment score is indicative of an overall post-therapy perceived severity of the therapy target.

5. The computer-implemented method of claim 1:
wherein generating the first one or more assessment scores includes generating a plurality of first category assessment scores, wherein each of the plurality of first category assessment scores is associated with a unique category of problem associated with the therapy target, and wherein each of the plurality of first category assessment scores is indicative of a strength of its respective unique category of problem;
wherein generating the second one or more assessment scores includes generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and
wherein training the assessment-based personalization model is based at least in part on the plurality of first category assessment scores, the plurality of second assessment scores, and the provided therapy.

6. The computer-implemented method of claim 1:
wherein generating the first one or more assessment scores includes generating a first overall assessment score, and wherein generating the first overall assessment score includes:
generating a plurality of first category assessment scores, wherein each of the plurality of first category assessment scores is associated with a unique category of problem associated with the therapy target, and wherein each of the plurality of first category assessment scores is indicative of a strength of its respective unique category of problem; and
calculating the first overall assessment score based at least in part on the plurality of first category assessment scores; and
wherein generating the second one or more assessment scores includes generating a second overall assessment score, and wherein generating the second overall assessment score includes:
generating a plurality of second category assessment scores, wherein each of the plurality of second category assessment scores is associated with a respect one of the plurality of first category assessment scores, and wherein each of the plurality of second category assessment scores is indicative of a strength of its respective unique category of problem; and
calculating the second overall assessment score based at least in part on the plurality of second category assessment scores.

7. The computer-implemented method of claim 1, wherein facilitating providing personalized therapy includes presenting a prompt to begin subsequent therapy using the determined subsequent therapy tool.

8. The computer-implemented method of claim 1:
wherein receiving the first user input data includes:
establishing, via a network interface, a chat interface with a user device associated with the user; and
receiving, via the chat interface, the first user input data;
wherein receiving the second user input data includes receiving, via the chat interface, the second user input data; and
wherein facilitating providing the personalized therapy includes initiating the subsequent therapy tool via the chat interface.

9. The computer-implemented method of claim 1, wherein the assessment-based personalization model is further trained using training data, wherein the training data is associated with a plurality of historical therapy sessions, and wherein, for each of the plurality of historical therapy sessions, the training data includes:
a historical first assessment score;
a historical second assessment score; and
a historical provided therapy associated with the historical therapy session.

10. The computer-implemented method of claim 1, wherein training the assessment-based personalization model includes accessing and further training an existing assessment-based personalization model, wherein the existing assessment-based personalization model is trained using training data associated with a cohort of users, the training data including pre-therapy assessment scores and post-therapy assessment scores associated with a plurality of historical provided therapy, and wherein the user is a member of the cohort of users.

11. The computer-implemented method of claim 1, further comprising receiving mood information associated with the user, wherein receiving the mood information includes receiving subsequent user input data and generating one or more mood scores based at least in part on the subsequent input data, wherein determining the subsequent therapy tool is further based on applying the one or more mood scores to a mood-based personalization model, wherein the mood-based personalization model is trained, at least in part, using training data that includes a plurality of historical mood scores associated with a plurality of historical therapy sessions provided to the user.

12. The computer-implemented method of claim 1, wherein facilitating providing the personalized therapy includes providing therapy to the user, and wherein providing the therapy to the user includes generating and transmitting a first message associated with the determined therapy tool.

13. A system, comprising:
one or more data processors; and
a non-transitory computer-readable storage medium containing instructions which, when executed on the one or more data processors, cause the one or more data processors to perform the method of any one of claims 1 to 12.

14. A computer-program product tangibly embodied in a non-transitory machine-readable storage medium, including instructions configured to cause a data processing apparatus to perform the method of any one of claims 1 to 12.
